# EUROPEAN PATENT APPLICATION

(11) **EP 4 534 071 A1**
(43) Date of publication of application: **09.04.2025**
(21) Application number: 23815949.5
(22) Date of filing: 26.05.2023
(51) Int. Cl.: A61K 8/81, A61K 8/34, A61K 8/41, A61K 8/73, A61K 8/86, A61K 8/891, A61K 8/894, A61K 8/898, A61Q 5/12

(54) **COSMETIC MATERIAL COMPOSITION AND FIBER TREATMENT COMPOSITION FOR HEAD DECORATION PRODUCTS**

(30) Priority: 30.05.2022 JP 2022087743; 23.03.2023 JP 2023046567
(71) Applicant: Kao Corporation, Tokyo 103-8210 (JP)
(72) Inventor: MIYOSHI Eisuke, Tokyo 131-8501 (JP); KAWAMOTO Koichi, Tokyo 131-8501 (JP); SARUKAWA Yuri, Tokyo 131-8501 (JP); MIENO Akiko, Tokyo 131-8501 (JP)
(74) Representative: Hoffmann Eitle
(86) International application number: PCT/JP2023/019641
(87) International publication number: WO 2023/234192

(57) **Abstract**

A cosmetic composition or a fiber treatment composition for head decoration products, the composition containing (A) a cationic polymer having a viscosity when in a 1 mass% aqueous solution form at 30°C of 1000 mPa·s or more, (B) an anionic polymer, (C) one or more modified silicones selected from the group consisting of a polyglycerol-modified silicone and an amino-modified silicone, (D) a dimethylpolysiloxane, (E) a cationic surfactant, and (F) a higher alcohol, wherein a mass ratio [(A)/(B)] of the component (A) to the component (B) is 4.5 or more.

## Description

### Technical Field

The present invention relates to a cosmetic composition or a fiber treatment composition for head decoration products.

### Background Art

Aqueous compositions for treating keratin materials containing cationic polymers and anionic polymers are known. For example, JP S53-139734 A (PTL 1) describes that using a composition for treating a keratin material, which is characterized by containing at least one anionic polymer and at least one cationic polymer in a solvent medium, makes it possible to fix the anionic polymer to a keratin material that can particularly include hair, skin, or nails. It is assumed that a complex formed by an interaction between the anionic polymer and the cationic polymer is related to the reason for the above-described effect.

Regarding hair cosmetics containing a cationic polymer and an anionic polymer, for example, JP 2008-297295 (PTL 2) discloses a hair treatment composition containing (A) L-theanine and (B) a nonionic polymer, an amphoteric polymer, and a cationic polymer as polymer compounds, and describes that the composition may contain an anionic polymer as necessary. It is disclosed that the hair treatment composition can improve elasticity in the vicinity of the hair root while imparting flexibility to the hair.

JP 2005-166333 (PTL 3) describes that a hair cosmetic containing a cationic polymer having a charge density in a predetermined range and an anionic polymer containing 80 mass% or more and 100 mass% or less of a predetermined anionic constituent unit and having a weight average molecular weight of 4000 or more and 80000 or less can improve the texture and appearance of damaged hair, and these effects can be maintained even by repeated washing of hair.

### Summary of Invention

The present invention relates to a cosmetic composition or a fiber treatment composition for head decoration products comprising (A) a cationic polymer having a viscosity when in a 1 mass% aqueous solution form at 30°C of 1000 mPa·s or more, (B) an anionic polymer, (C) one or more modified silicones selected from the group consisting of a double-terminal hydroxy-modified silicone, a polyglycerol-modified silicone, an amino-modified silicone, an aminopolyether-modified silicone, and a polyether-modified silicone, (D) a dimethylpolysiloxane, (E) a cationic surfactant, and (F) a higher alcohol, wherein a mass ratio [(A)/(B)] of the component (A) to the component (B) is 4.5 or more.

### Description of Embodiments

As described in PTL 1, it is known to use a composition containing a complex formed from a cationic polymer and an anionic polymer (hereinafter, also referred to as "polyion complex") as a composition for treating a keratin substance such as hair, but there is still room for improvement in terms of performance improvement. For example, in a hair conditioner which is a type of hair cosmetic composition, it is required that the texture of hair after the hair conditioner is applied is good, entanglement of the hair is suppressed, and the hair after treatment does not spread even under high humidity conditions, and the hair has excellent moisture resistance. However, no particular effect has been obtained in the known technologies according to PTLs 1 to 3. In addition, the hair treatment effect may be lost by one time of hair washing, and the durability (washing resistance) of the treatment effect has been desired.

Further, as a composition containing a polyion complex, there is a composition in the form of an oil-in-water type or a water-in-oil type emulsion composition, but in these emulsion compositions, improvement in stability with time has been a problem. In particular, in a hair cosmetic composition, a cationic surfactant, a higher alcohol, or the like may be blended in order to improve the texture of hair after treatment. However, there has been a problem that when these components are blended in an emulsion composition containing a polyion complex, the stability is likely to be lowered.

The present invention relates to a hair cosmetic composition or a fiber treatment composition for head decoration products that can improve a texture improving effect of an object to be treated, durability of the effect, and moisture resistance, and has high stability.

The present inventors have found that the above problem can be solved by a composition containing a cationic polymer having a viscosity when in a 1 mass% aqueous solution form of a predetermined value or more, an anionic polymer, a predetermined silicone compound, and water.

The present invention can provide a cosmetic composition or a fiber treatment composition for head decoration products that can improve the texture improving effect of an object to be treated, the durability of the effect, and moisture resistance. Especially when these compositions are used for treating hair or fibers for head decoration products, the composition can improve the effect of suppressing entanglement of hair or fibers for head decoration products and the durability thereof, can suppress the spread of hair under high humidity conditions, and has high stability.

### [Cosmetic Composition or Fiber Treatment Composition for Head Decoration Product]

A cosmetic composition or a fiber treatment composition for head decoration products of the present invention (hereinafter, may be simply referred to as "composition of the present invention") contains (A) a cationic polymer having a viscosity when in a 1 mass% aqueous solution form at 30°C of 1000 mPa·s or more, (B) an anionic polymer, (C) one or more modified silicones selected from the group consisting of a polyglycerol-modified silicone and an amino-modified silicone, (D) a dimethylpolysiloxane, (E) a cationic surfactant, and (F) a higher alcohol, wherein a mass ratio [(A)/(B)] of the component (A) to the component (B) is 4.5 or more.

In the present specification, "containing a component X" also includes blending of the component X.

The composition of the present invention having the above configuration can improve the texture improving effect of the object to be treated, the durability of the effect, and moisture resistance, and particularly when the composition is used for treating hair or fibers for head decoration products, the composition can improve the effect of suppressing entanglement of hair and the durability of the effect, can suppress spreading of hair under high humidity conditions, and has high stability.

The reason for the composition of the present invention exhibiting the effects described above is not clear, but the reason is thought to be as follows.

The composition of the present invention contains a polyion complex formed from the component (A) and the component (B) in a predetermined ratio. In the polyion complex, a crosslinked structure is formed by an ionic interaction between the component (A) and the component (B), and it is assumed that this structure causes the film obtained by using the composition of the present invention to exhibit a high elastic modulus. Further, the polyion complex is poorly water-soluble, and a hydrophobic film can be formed by applying a composition containing the polyion complex to an object. It is considered that the formation of the hydrophobic film improves the texture and moisture resistance of the object to be treated.

The component (A) used in the present invention has a viscosity when in a 1 mass% aqueous solution form at 30°C of 1000 mPa·s or more. It is considered that using the component (A) improves the lubricity of the obtained film, which further improves the texture of the object to be treated, and when the composition of the present invention is used for treating hair or fibers for head decoration products, the effect of suppressing entanglement of hair or fibers for head decoration products improves. In addition, it is considered that the structure of the polyion complex is hardly destroyed and the stability is improved even when an oily component such as the component (E) or (F) is added to form an emulsion composition, since the polyion complex containing the component (A) has high stability.

The composition of the present invention further contains silicone compounds as components (C) and (D). The modified silicone as the component (C) has high adsorptivity to keratin materials, and the component (D) is a compound having high hydrophobicity and low surface tension. Thus, it is considered that when the composition of the present invention is applied to an object to be treated, the surface of the object is hydrophobically coated by the components (C) and (D), and the texture improving effect of the object to be treated, the durability of the effect, and the moisture resistance further improve.

The composition of the present invention is preferably a cosmetic composition for keratin materials such as skin, hair, eyelashes, eyebrows, and nails, or a fiber treatment composition for head decoration products, more preferably a cosmetic composition for skin or hair, and a fiber treatment composition for head decoration products, even more preferably a cosmetic composition for skin or hair, and even more preferably a hair cosmetic composition.

Among the cosmetic compositions, examples of the product form of the skin cosmetic composition include a body soap, a facial cleanser, a bath agent, a deodorant preparation, a makeup cosmetic, a sunscreen, a makeup foundation, a milky lotion, a beauty essence, and a cream.

Examples of the product form of the hair cosmetic composition include a hair shampoo, a hair rinse, a hair conditioner, a hair treatment (including a leave-on type), a hair styling agent, a hair coloring agent, and a permanent agent. Among these, from the viewpoint of the effectiveness of the effects of the present invention, a hair conditioner, a hair treatment, or a hair styling agent is preferable.

The fibers for head decoration products to which the composition of the present invention is applied may be either naturally derived fibers or synthetic fibers, but naturally derived fibers are preferable. The naturally derived fibers refer to fibers collected from a natural plant or animal, or fibers artificially produced using collagen, casein, soybean, peanut, corn, silk waste silk fibroin, or the like as a raw material and used for a head decoration product. Among these, fibers artificially produced from collagen, casein, soybean, peanut, corn, silk waste silk fibroin, or the like as a raw material are preferable, regenerated protein fibers such as regenerated collagen fibers using collagen as a raw material and regenerated silk fibers using silk fibroin as a raw material are more preferable, and regenerated collagen fibers are even more preferable.

Regenerated collagen fibers can be produced by known techniques. The composition of the regenerated collagen fibers does not need to be 100% collagen, and the composition may contain natural polymers, synthetic polymers, additives, and the like for quality improvement. Further, the regenerated collagen fibers may be post-processed.

The regenerated collagen fibers are preferably in the form of filaments. The filaments are generally taken from a bobbin wound or boxed condition. It is also possible to directly utilize the filaments coming out of the drying step in the production process of regenerated collagen fibers.

Examples of the synthetic fiber include a fiber containing a synthetic resin as a main component. The synthetic resin is preferably a thermoplastic resin, and more preferably one or more selected from the group consisting of polyester resins, polyamide resins, polyimide resins, polyamide-imide resins, vinyl chloride resins, polycarbonate resins, polyphenylene sulfide resins, and modacrylic resins (copolymer of acrylonitrile and vinyl chloride), from the viewpoint of ease of production of the synthetic fiber and from the viewpoint of obtaining a feeling close to that of hair. The term "main component" as used herein means a component whose content in the synthetic fiber is preferably 50 mass% or more, more preferably 60 mass% or more, even more preferably 70 mass% or more, even more preferably 80 mass% or more, and even more preferably 90 mass% or more, and is 100 mass% or less.

The synthetic fiber may further contain, in addition to the above-described synthetic resin, various additives such as a flame retardant, a flame retardant aid, a light or heat stabilizer, a fluorescent agent, an antioxidant, an antistatic agent, and an ultraviolet absorber, as long as the effects of the present invention are not impaired.

The form of the composition of the present invention is preferably an emulsion composition, and more preferably an oil-in-water emulsion composition from the viewpoint of improving the stability of the composition and improving the feeling of use.

Hereinafter, each component contained in the composition of the present invention will be described.

### (Component (A): Cationic Polymer)

The component (A) is a cationic polymer having a viscosity when in a 1 mass% aqueous solution form at 30°C of 1000 mPa·s or more, and is a polymer capable of forming a polyion complex through interaction with the component (B).

The cationic polymer as the component (A) is preferably a polymer having a cationic group and being positively charged as a total charge. The component (A) may have an anionic group, a nonionic group, or an amphoteric group such as a betaine group in addition to the cationic group as long as the effects of the present invention are not impaired.

In the present specification, the cationic group is a cationic group or a group that can be ionized to become a cationic group, and specific examples thereof include a primary amino group, a secondary amino group, a tertiary amino group, and a quaternary ammonium group.

The anionic group is an anionic group or a group that can be ionized to become an anionic group, and specific examples thereof include one or more selected from the group consisting of acidic groups such as a carboxyl group, a sulfonic acid group, and a phosphoric acid group, preferably one or more selected from a carboxyl group and a sulfonic acid group, and more preferably a carboxyl group. At least a part of the anionic groups may be neutralized to form a salt.

The viscosity of a 1 mass% aqueous solution of the component (A) at 30°C is 1000 mPa·s or more, preferably 1300 mPa·s or more, more preferably 1500 mPa·s or more, even more preferably 1700 mPa·s or more, and even more preferably 1800 mPa·s or more, from the viewpoint of the texture improving effect of the object to be treated and the improvement of the durability of the effect, in particular, the effect of suppressing entanglement of hair or fibers for head decoration products when used for treating hair or fibers for head decoration products and the improvement of the durability thereof, the improvement of the moisture resistance, and from the viewpoint of the improvement of the stability of the composition. The upper limit of the viscosity is not particularly limited, but is preferably 100000 mPa·s or less, more preferably 80000 mPa·s or less, even more preferably 50000 mPa·s or less, even more preferably 30000 mPa·s or less, and even more preferably 25000 mPa·s or less, from the viewpoint of ease of forming a polyion complex and handleability. The viscosity of a 1 mass% aqueous solution of the component (A) at 30°C is 1000 mPa·s or more, preferably 1000 mPa·s or more and 100000 mPa·s or less, more preferably 1300 mPa·s or more and 80000 mPa·s or less, even more preferably 1500 mPa·s or more and 50000 mPa·s or less, even more preferably 1700 mPa·s or more and 30000 mPa·s or less, and even more preferably 1800 mPa·s or more and 25000 mPa·s or less.

The viscosity of a 1 mass% aqueous solution of the component (A) at 30°C can be specifically measured by the method described in Examples.

The cationic charge density of the component (A) is preferably 0.1 mmol/g or more, more preferably 0.2 mmol/g or more, even more preferably 0.3 mmol/g or more, and even more preferably 0.5 mmol/g or more, from the viewpoint of facilitating formation of a polyion complex through interaction with the component (B), from the viewpoints of the texture improving effect of the object to be treated and the improvement of the durability of the effect, in particular, the effect of suppressing entanglement of hair or fibers for head decoration products when used for treating hair or fibers for head decoration products and the improvement of the durability thereof, from the viewpoint of the improvement of moisture resistance, and from the viewpoint of the improvement of the stability of the composition. The cationic charge density is preferably 20 mmol/g or less, more preferably 15 mmol/g or less, even more preferably 12 mmol/g or less, even more preferably 10 mmol/g or less, even more preferably 7.0 mmol/g or less, and even more preferably 5.0 mmol/g or less from the viewpoints of the texture improving effect of the object to be treated and the improvement of the durability of the effect, in particular, the effect of suppressing entanglement of hair or fibers for head decoration products when used for treating hair or fibers for head decoration products and the improvement of the durability thereof, from the viewpoint of the improvement of the moisture resistance, and from the viewpoint of the improvement of the stability of the composition. The cationic charge density of the component (A) is preferably 0.1 mmol/g or more and 20 mmol/g or less, more preferably 0.2 mmol/g or more and 15 mmol/g or less, 0.2 mmol/g or more and 12 mmol/g or less, even more preferably 0.3 mmol/g or more and 10 mmol/g or less, even more preferably 0.3 mmol/g or more and 7.0 mmol/g or less, and even more preferably 0.5 mmol/g or more and 5.0 mmol/g or less.

The cationic charge density of the component (A) is the number of moles of cationic groups contained per polymer 1 g. When at least a part of the cationic groups of the component (A) is in the form of a neutralized salt, the number of moles of the cationic groups includes the number of moles of the cationic groups in the form of a salt.

Two or more polymers may be used as the component (A), and in this case, the cationic charge density of the component (A) is determined by calculating a weighted average from the cationic charge density and the blending amount of each polymer.

As the component (A), any of a natural polymer such as a cationized polysaccharide or a cationized product thereof and a synthetic polymer can be used, but from the viewpoint of availability, a synthetic polymer is preferable.

Specific examples of the polymer used as the component (A) include cationized polyvinyl alcohols, polyethyleneimines, methacryloylethyltrimethylammonium salt polymers, quaternized dialkylaminoalkyl (meth)acrylate polymers, diallylquaternized ammonium salts, methacrylamidopropyltrimethylammonium salt polymers, vinylimidazoliumtrichloride-vinylpyrrolidone copolymers (polyquaternium-16), vinylpyrrolidone-alkylamino (meth)acrylate copolymers, vinylpyrrolidone-alkylamino (meth)acrylate-vinylcaprolactam copolymers, alkylacrylamide-(meth)acrylate-alkylaminoalkylacrylamide-polyethylene glycol (meth)acrylate copolymers, and adipic acid-dimethylaminohydroxypropylethylenetriamine copolymers having a viscosity when in a 1 mass% aqueous solution form at 30°C of 1000 mPa·s or more, and one or two or more of these can be used. In the present specification, "(meth)acrylic acid" means acrylic acid or methacrylic acid, and "(meth)acrylate" means acrylate or methacrylate.

Among these, examples of the methacryloylethyltrimethylammonium salt polymer include a methacryloylethyltrimethylammonium chloride polymer (polyquaternium-37), a methacryloylethyldimethylbetaine-methacryloylethyltrimethylammonium chloride-methoxypolyethylene glycol methacrylate copolymer (polyquaternium-49), and a methacryloylethyldimethylbetaine-methacryloylethyltrimethylammonium chloride-2-hydroxyethyl methacrylate copolymer (polyquaternium-48).

Examples of the quaternized dialkylaminoalkyl (meth)acrylate polymer include a vinylpyrrolidone-N,N-dimethylaminoethyl methacrylate diethyl sulfate copolymer (polyquaternium-11) and an N,N-dimethylaminoethyl methacrylate diethyl sulfate-N,N-dimethylacrylamide-polyethylene glycol dimethacrylate copolymer (polyquaternium-52).

Examples of the diallyl quaternized ammonium salt polymer include a dimethyldiallylammonium chloride polymer (polyquaternium-6), a dimethyldiallylammonium chloride-acrylic acid copolymer (polyquaternium-22), a dimethyldiallylammonium chloride-acrylamide copolymer (polyquaternium-7), and an acrylamide-acrylic acid-dimethyldiallylammonium chloride copolymer (polyquaternium-39).

Examples of the methacrylamidopropyltrimethylammonium salt polymer include a methacrylamidopropyltrimethylammonium chloride polymer, a vinylpyrrolidone-methacrylamidopropyltrimethylammonium chloride copolymer, an acrylic acid-methyl acrylate-methacrylamidopropyltrimethylammonium chloride copolymer (polyquaternium-47), and an acrylic acid-acrylamide-methacrylamidopropyltrimethylammonium chloride copolymer (polyquaternium-53).

The component (A) is preferably a polymer containing a constituent unit represented by the following General Formula (1) from the viewpoint of improving the film-forming ability, from the viewpoints of the texture improving effect of the object to be treated and the improvement of the durability of the effect, in particular, the effect of suppressing entanglement of hair or fibers for head decoration products when used for treating hair or fibers for head decoration products and the improvement of the durability thereof, and from viewpoint of the improvement of the moisture resistance. 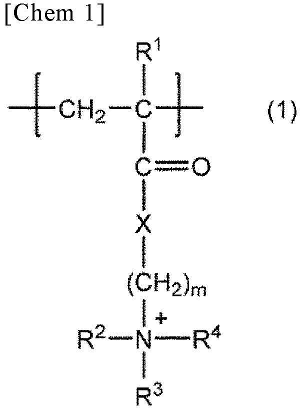 wherein R¹ is a hydrogen atom or a methyl group, and R² to R⁴ are each independently an alkyl group having 1 or more and 4 or less carbons, X is -O- or -NH-, and m is the number of 1 to 4.

In Formula (1), R¹ is preferably a methyl group, and R² to R⁴ are each independently preferably an alkyl group having 1 or more and 3 or less carbons, more preferably a methyl group or an ethyl group, and even more preferably a methyl group.

X in Formula (1) is preferably -O-, and m is preferably 1 or more and 3 or less, and more preferably 2 or more and 3 or less.

Examples of the constituent unit represented by Formula (1) include one or more selected from the group consisting of a constituent unit derived from a methacryloylethyltrimethylammonium salt, a constituent unit derived from N,N-dimethylaminoethylmethacrylic acid diethyl sulfate, and a constituent unit derived from a methacrylamidopropyltrimethylammonium salt, and preferably one or more selected from the group consisting of a constituent unit derived from a methacryloylethyltrimethylammonium salt and a constituent unit derived from N,N-dimethylaminoethylmethacrylic acid diethyl sulfate.

The component (A) may be a polymer containing an additional constituent unit other than the constituent unit represented by Formula (1). Examples of the additional constituent unit include constituent units derived from vinyl monomers such as vinylpyrrolidone, amphoteric monomers such as (meth)acryloylethyldimethylbetaine, hydroxyalkyl (meth)acrylates such as (meth)acrylic acid alkyl esters and 2-hydroxyethyl (meth)acrylate, and acrylamides such as N,N-dimethylacrylamide. The component (A) may also be a crosspolymer crosslinked with di(meth)acrylic acid polyethylene glycol or the like.

**In** the component (A), the content of the constituent unit represented by Formula (1) is preferably 8.0 mol% or more, more preferably 10 mol% or more, and is 100 mol% or less, based on all constituent units constituting the component (A), from the viewpoint of improving the film-forming ability, from the viewpoints of the texture improving effect of the object to be treated and the improvement of the durability of the effect, in particular, the effect of suppressing entanglement of hair or fibers for head decoration products when used for treating hair or fibers for head decoration products, and the improvement of the durability thereof.

The component (A) may be used alone or in combination of two or more thereof. Among the above, the component (A) is preferably one or more selected from the group consisting of a methacryloyl ethyltrimethylammonium salt polymer and a quaternary dialkylaminoalkyl (meth)acrylate salt polymer, more preferably one or more selected from the group consisting of a methacryloyl ethyltrimethylammonium chloride polymer (polyquaternium-37), a methacryloyl ethyldimethylbetaine-methacryloyl trimethylammonium chloride-methacrylic acid methoxy polyethylene glycol copolymer (polyquaternium-49), a methacryloyl ethyl dimethyl betaine-methacryloyl ethyl trimethyl ammonium chloride-2-hydroxyethyl methacrylate copolymer (polyquaternium-48) and an N,N-dimethylaminoethyl methacrylic acid diethyl sulfate-N,N-dimethylacrylamide-polyethylene glycol dimethacrylate copolymer (polyquaternium-52), and even more preferably one or more selected from the group consisting of a methacryloyl ethyl trimethyl ammonium chloride polymer (polyquaternium-37) and an N,N-dimethylaminoethyl methacrylic acid diethyl sulfate-N,N-dimethylacrylamide-polyethylene glycol dimethacrylate copolymer (polyquaternium-52), from the viewpoint of easily setting the viscosity when in a 1 mass% aqueous solution form at 30°C to a value equal to or larger than a predetermined value, from the viewpoints of the texture improving effect of the object to be treated and the improvement of the durability of the effect, in particular, the effect of suppressing entanglement of hair or fibers for head decoration products when used for treating hair or fibers for head decoration products and the improvement of the durability of the effect, from the viewpoint of the improvement of the moisture resistance, and from the viewpoint of the improvement of the stability of the composition.

As the component (A), a commercially available polymer can also be used. Specific examples thereof include "Cosmedia Ultragel 300" (polyquaternium-37, cationic charge density: 4.81 mmol/g) manufactured by BASF Japan Ltd., "SOFCARE KG-101W-E" (polyquaternium-52, cationic charge density: 0.83 mmol/g), and "SOFCARE KG-301W" (polyquaternium-52, cationic charge density: 1.84 mmol/g) manufactured by Kao Corporation.

### (Component (B): Anionic Polymer)

The component (B) is a polymer capable of forming a polyion complex through interaction with the component (A). In the present specification, "anionic polymer" means a polymer having an anionic group and substantially having no cationic group and no amphoteric group. "Having substantially no cationic group and no amphoteric group" means that the molar amount of the cationic group and the amphoteric group relative to the anionic group is preferably 0.1% or less.

The anionic group of the component (B) is preferably an acidic group such as a carboxy group, a sulfonic acid group, or a phosphoric acid group, and is more preferably a carboxy group from the viewpoint of facilitating formation of a polyion complex through interaction with the component (A) and from the viewpoint of availability. In the component (B), at least a part of the anionic groups may be neutralized to form a salt.

### <Component (B1): Crosslinked Polymer Containing (Meth)Acrylic Acid-Derived Constituent Unit, and Anionic Polysaccharides>

The component (B) preferably contains (B1) one or more selected from the group consisting of a cross-linked polymer containing a (meth)acrylic acid-derived constituent unit and an anionic polysaccharide, from the viewpoint of the texture improving effect of the object to be treated and the improvement of the durability of the effect, in particular, from the viewpoint of the improvement of the effect of suppressing entanglement of hair or fibers for head decoration products when used for treating hair or fibers for head decoration products and the improvement of the durability thereof, from the viewpoint of the improvement of the moisture resistance, and from the viewpoint of the improvement of the stability of the composition.

Among the components (B 1), examples of the cross-linked polymer containing a constituent unit derived from (meth)acrylic acid include homopolymer or a copolymer of (meth)acrylic acid having a crosslinked structure, and a salt thereof. The term "crosslinked structure" as used herein refers to a three-dimensional network structure in which polymer chains, which are the main structure of a polymer, are linked within a polymer chain or between polymer chains. The method for forming the cross-linked structure is not particularly limited, and examples thereof include a method of cross-linking simultaneously with polymerization such as polycondensation or radical polymerization, and a method of cross-linking polymer chains afterwards.

Examples of the homopolymer of (meth)acrylic acid include a polyacrylic acid, a polymethacrylic acid, and a salt thereof.

Examples of the copolymer of (meth)acrylic acid include a (meth)acrylic acid/maleic acid copolymer, a (meth)acrylic acid/itaconic acid copolymer, a (meth)acrylic acid/fumaric acid copolymer, a (meth)acrylic acid/vinyl acetate copolymer, a (meth)acrylic acid/(meth)acrylic acid alkyl ester copolymer, a (meth)acrylic acid/2-hydroxyethyl methacrylate copolymer, an acrylic acid/acrylic acid alkyl ester/(N-alkyl)acrylamide copolymer, and a salt thereof, and one or two or more of these copolymers can be used.

Among these, as the copolymer of (meth)acrylic acid, a (meth)acrylic acid/(meth)acrylic acid alkyl ester is preferable, and an acrylic acid/acrylic acid alkyl ester is more preferable. Examples of the (meth)acrylic acid alkyl ester include (meth)acrylic acid alkyl esters in which the number of carbons of the alkyl is preferably 1 or more, more preferably 4 or more, and even more preferably 8 or more, and is preferably 40 or less, more preferably 36 or less, and even more preferably 32 or less.

Specific examples of the cross-linked polymer containing a constituent unit derived from (meth)acrylic acid include a carboxyvinyl polymer or a salt thereof, an (acrylates/alkyl acrylate (C10-30)) crosspolymer, a (Na acrylate/acryloyldimethyltaurine/dimethylacrylamide) crosspolymer, and an acrylates crosspolymer-4.

Among the components (B 1), examples of the anionic polysaccharides include polysaccharides having a carboxyl group (hyaluronic acid, alginic acid, pectic acid, carboxymethyl cellulose, xanthan gum, and the like), sulfates of polysaccharides (carrageenan, keratan sulfate, dermatan sulfate, sulfated starch, heparin, heparan sulfate), and a salt thereof, and one or two or more of these can be used.

Among the above, the anionic polysaccharide is preferably a polysaccharide having a carboxyl group or a salt thereof, more preferably alginic acid, carboxymethyl cellulose, or a salt thereof, and even more preferably alginic acid or a salt thereof, from the viewpoint of the texture improving effect of the object to be treated and the improvement of the durability of the effect, in particular, the effect of suppressing entanglement of hair or fibers for head decoration products when used for treating hair or fibers for head decoration products and the improvement of the durability thereof.

When the component (B1) is a salt, examples of the salt include alkali metal salts such as a sodium salt and a potassium salt, and alkaline earth metal salts, and from the viewpoint of availability, an alkali metal salt is preferable.

Among the above, the component (B1) is preferably one or more selected from the group consisting of a carboxyvinyl polymer, an (acrylates/alkyl acrylate (C10-30)) cross polymer, and an anionic polysaccharide, more preferably an anionic polysaccharide or a salt thereof, and even more preferably alginic acid or a salt thereof, from the viewpoint of the texture improving effect of the object to be treated and the improvement of the durability of the effect, in particular, the effect of suppressing entanglement of hair or fibers for head decorative products when used for treating hair or fibers for head decorative products and the improvement of the durability thereof, from the viewpoint of the improvement of the moisture resistance, and from the viewpoint of the improvement of the stability of the composition.

The anionic charge density of the component (B1) is not particularly limited, but is preferably 1.0 mmol/g or more, more preferably 2.0 mmol/g or more, and even more preferably 3.5 mmol/g or more from the viewpoint of facilitating formation of a polyion complex through interaction with the component (A), from the viewpoint of the texture improving effect of the object to be treated and the improvement of the durability of the effect, in particular, the effect of suppressing entanglement of hair or fibers for head decoration products when used for treating hair or fibers for head decoration products and the improvement of the durability thereof, from the viewpoint of the improvement of the moisture resistance, and from the viewpoint of the improvement of the stability of the composition. The charge density is preferably 25 mmol/g or less, more preferably 20 mmol/g or less, and even more preferably 15 mmol/g or less, from the viewpoint of the texture improving effect of the object to be treated and the improvement of the durability thereof, in particular, the effect of suppressing entanglement of hair or fibers for head decoration products when used for treating hair or fibers for head decoration products and the improvement of the durability thereof. The anionic charge density of the component (B1) is preferably 1.0 mmol/g or more and 25 mmol/g or less, more preferably 2.0 mmol/g or more and 20 mmol/g or less, even more preferably 3.5 mmol/g or more and 15 mmol/g or less.

The anionic charge density of the component (B1) is the number of moles of anionic groups contained per polymer 1 g. When at least a part of the anion groups of the component (B1) is in the form of a neutralized salt, the number of moles of anion groups includes the number of moles of anion groups in the form of a salt.

Two or more polymers may be used as the component (B1), and in this case, the anionic charge density of the component (B1) is determined by calculating a weighted average from the anionic charge density and the blending amount of each polymer.

When the component (B1) is an anionic polysaccharide, the weight average molecular weight (Mw) thereof is preferably 2000 or more, more preferably 10000 or more, even more preferably more than 30000, and even more preferably 50000 or more from the viewpoint of improving the film-forming ability. The weight average molecular weight is preferably 3500000 or less, and more preferably 3000000 or less from the viewpoint of facilitating the formation of a polyion complex through the interaction with the component (A), from the viewpoints of the texture improving effect of the object to be treated and the improvement of the durability of the effect, in particular, the effect of suppressing entanglement of hair or fibers for head decoration products when used for treating hair or fibers for head decoration products and the improvement of the durability, from the viewpoint of the improvement of moisture resistance, and from the viewpoint of the improvement of the stability of the composition. The weight average molecular weight (Mw) of the anionic polysaccharide as the component (B1) is preferably 2000 or more and 3500000 or less, more preferably 10000 or more and 3000000 or less, even more preferably more than 30000 and 3000000 or less, and even more preferably 50000 or more and 3000000 or less.

The weight average molecular weight of the component (B1) can be measured by gel permeation chromatography (GPC).

The content of the component (B1) in the component (B) is preferably 50 mass% or more, more preferably 60 mass% or more, still more preferably 70 mass% or more, even still more preferably 80 mass% or more, and is 100 mass% or less from the viewpoint of improving the film-forming ability, from the viewpoint of easily forming a polyion complex through interaction with the component (A), from the viewpoint of the texture improving effect of the object to be treated and the improvement of the durability of the effect, in particular, from the viewpoint of the improvement of the effect of suppressing entanglement of hair or fibers for head decoration products when used for treating hair or fibers for head decoration products and the improvement of the durability thereof, from the viewpoint of the improvement of the moisture resistance, and from the viewpoint of the improvement of the stability of the composition.

### <Component (B2): Anionic Polymer Other Than Component (B1)>

The component (B) preferably further contains (B2) an anionic polymer other than the component (B1) from the viewpoint of further improving the texture improving effect of the object to be treated and the durability of the effect, in particular, from the viewpoint of the effect of suppressing entanglement of hair or fibers for head decoration products when used in for treating hair or fibers for head decoration products and the improvement of the durability thereof, and from the viewpoint of the improvement of the moisture resistance.

When the composition of the present invention further contains (G) a nonionic polymer described later, it is considered that the component (B2) hydrogen-bonds with (G) a nonionic polymer, and the component (G) can be immobilized in the crosslinked structure of the polyion complex formed by the component (A)-the component (B). It is considered that due to this effect, even when rinsing or washing is performed after treating an object to be treated with the composition of the present invention, the component (G) is not washed away and is likely to remain in the film, and the durability of the treatment effect is improved.

The molecular weight of the component (B2) is preferably 1000 or more, more preferably 2000 or more, even more preferably 5000 or more, even more preferably 10000 or more, and preferably 50000 or less, more preferably 40000 or less, even more preferably 30000 or less from the viewpoint of contributing to the immobilization of the component (G) to further improve the texture improving effect of the object to be treated and the durability of the effect, in particular, from the viewpoint of the effect of suppressing entanglement of hair or fibers for head decoration products when used for treating hair or fibers for head decoration products and the improvement of the durability thereof, and from the viewpoint of the improvement of the moisture resistance. The molecular weight of the component (B2) is preferably 1000 or more and 50000 or less, more preferably 1000 or more and 30000 or less, even more preferably 2000 or more and 30000 or less, even more preferably 5000 or more and 30000 or less, and even more preferably 10000 or more and 30000 or less.

The term "molecular weight" as used herein means a weight average molecular weight and can be measured by gel permeation chromatography (GPC).

The component (B2) is preferably a polymer having a carboxy group with a molecular weight of 1000 or more and 50000 or less, more preferably 1000 or more and 30000 or less, and more preferably a non-crosslinked polymer containing a constituent unit derived from (meth)acrylic acid with a molecular weight of 1000 or more and 30000 or less.

Examples of the non-crosslinked polymer containing a constituent unit derived from (meth)acrylic acid suitably used as the component (B2) include, in addition to poly(meth)acrylic acid, a (meth)acrylic acid/maleic acid copolymer, a (meth)acrylic acid/itaconic acid copolymer, a (meth)acrylic acid/fumaric acid copolymer, a (meth)acrylic acid/vinyl acetate copolymer, a (meth)acrylic acid/(meth)acrylic acid alkyl ester copolymer, a (meth)acrylic acid/2-hydroxyethyl methacrylate copolymer, an acrylic acid/acrylic acid alkyl ester/(N-alkyl)acrylamide copolymer, and a salt thereof, and one or two or more of these can be used.

Examples of the polymer having a carboxy group other than those described above and suitably used as the component (B2) include polymaleic acid, a maleic acid/diisobutylene copolymer, a maleic acid/styrene copolymer, a benzoic acid formaldehyde condensate, a benzoic acid/phenol/formaldehyde condensate, and a salt thereof.

Among the above, the component (B2) is preferably one or more selected from the group consisting of a poly(meth)acrylic acid, a (meth)acrylic acid/(meth)acrylic acid alkyl ester copolymer, and a salt thereof from the viewpoint of further improving the texture improving effect of the object to be treated and the durability of the effect, in particular, from the viewpoint of the effect of suppressing entanglement of hair or fibers for head decoration products when used for treating hair or fibers for head decoration products and the improvement of the durability thereof, and from the viewpoint of the improvement of the moisture resistance.

Examples of the poly(meth)acrylic acid or a salt thereof include polyacrylic acid, polymethacrylic acid, and a salt thereof, and polyacrylic acid or a salt thereof is preferable.

As the (meth)acrylic acid/(meth)acrylic acid alkyl ester copolymer or a salt thereof, an acrylic acid/acrylic acid alkyl ester or a salt thereof is more preferable. Examples of the (meth)acrylic acid alkyl ester include a (meth)acrylic acid alkyl ester in which the number of carbons in the alkyl is preferably 1 or more, more preferably 4 or more, even more preferably 8 or more, even more preferably 12 or more, and preferably 40 or less, more preferably 36 or less, even more preferably 32 or less, even more preferably 24 or less.

The (meth)acrylic acid/(meth)acrylic acid alkyl ester is preferably an acrylic acid/acrylic acid alkyl ester, more preferably one or more selected from the group consisting of an (acrylic acid/octyl acrylate) copolymer and an (acrylic acid/stearyl acrylate) copolymer, and even more preferably an (acrylic acid/stearyl acrylate) copolymer.

When the component (B2) is a salt, examples of the salt include alkali metal salts such as a sodium salt and a potassium salt, and alkaline earth metal salts, and from the viewpoint of availability, an alkali metal salt is preferable.

The anionic charge density of the component (B2) is not particularly limited, but is preferably 1.0 mmol/g or more, more preferably 3.0 mmol/g or more, and even more preferably 5.0 mmol/g or more, and is preferably 25 mmol/g or less, more preferably 20 mmol/g or less, and even more preferably 15 mmol/g or less from the viewpoint of further improving the texture improving effect of the object to be treated and the durability of the effect, in particular, from the viewpoint of the effect of suppressing entanglement of hair or fibers for head decoration products when used for treating hair or fibers for head decoration products and the improvement of the durability thereof, and from the viewpoint of the improvement of the moisture resistance. The anionic charge density of the component (B2) is preferably 1.0 mmol/g or more and 25 mmol/g or less, more preferably 3.0 mmol/g or more and 20 mmol/g or less, even more preferably 5.0 mmol/g or more and 15 mmol/g or less.

Two or more polymers may be used as the component (B2), and in this case, the anionic charge density of the component (B2) is determined by calculating a weighted average from the anionic charge density and the blending amount of each polymer.

When the component (B) contains the component (B2), the content of the component (B2) in the component (B) is preferably 1 mass% or more, more preferably 2 mass% or more, even more preferably 3 mass% or more, and even more preferably 5 mass% or more from the viewpoint of further improving the texture improving effect of the object to be treated and the durability of the effect, in particular, from the viewpoint of the effect of suppressing entanglement of hair or fibers for head decoration products when used for treating hair or fibers for head decoration products and the improvement of the durability of the effect, and from the viewpoint of the improvement of the moisture resistance, and the content is preferably 50 mass% or less, more preferably 40 mass% or less, even more preferably 30 mass% or less, and even more preferably 20 mass% or less from the viewpoint of ease of forming a polyion complex with the component (A). The content of the component (B2) in the component (B) is preferably 1 mass% or more and 50 mass% or less, more preferably 2 mass% or more and 40 mass% or less, even more preferably 3 mass% or more and 30 mass% or less, and even more preferably 5 mass% or more and 20 mass% or less.

When the component (B) contains the component (B1) and the component (B2), the mass ratio of the component (B2) to the total amount of the component (B1) and the component (B2) [(B2)/{(B1) + (B2)}] is preferably 0.01 or more, more preferably 0.02 or more, even more preferably 0.05 or more, and even more preferably 0.08 or more from the viewpoint of further improving the texture improving effect of the object to be treated and the durability of the effect, in particular, from the viewpoint of the effect of suppressing entanglement of hair or fibers for head decoration products when used for treating hair or fibers for head decoration products and the improvement of the durability of the effect, and from the viewpoint of the improvement of the moisture resistance. From the viewpoint of easily forming a polyion complex with the component (A), the mass ratio is preferably 1.0 or less, more preferably 0.80 or less, even more preferably 0.50 or less, and even more preferably 0.30 or less. The mass ratio [(B2)/{(B1) + (B2)}] is preferably 0.01 or more and 1.0 or less, more preferably 0.02 or more and 0.80 or less, even more preferably 0.05 or more and 0.50 or less, and even more preferably 0.08 or more and 0.30 or less.

### (Component (C): Modified Silicone)

The component (C) is one or more modified silicones selected from the group consisting of polyglycerol-modified silicones and amino-modified silicones. It is considered that containing the component (C) in the composition improves the texture improving effect of the object to be treated and the durability of the effect, in particular, the effect of suppressing the entanglement of hair or fibers for head decoration products when used for treating hair or fibers for head decoration products and the durability thereof, and the moisture resistance.

### [Polyglycerol-Modified Silicone]

The polyglycerol-modified silicone is a silicone having a polyglycerol chain in the molecule. The position at which the polyglycerol chain is introduced is any position, and the form of introduction may be any of a single-terminal type, a both-terminal type, a side-chain type, and the like, but a silicone having a polyglycerol chain in a side chain or at a terminal of a silicone chain is preferable, and a silicone having a monovalent polyglyceryl group in a side chain or at a terminal of a silicone chain is more preferable.

Specific examples of the polyglycerol-modified silicone include polyglyceryl-3 polydimethylsiloxyethyl dimethicone, polyglyceryl-3 disiloxane dimethicone, lauryl polyglyceryl-3 polydimethylsiloxyethyl dimethicone, cetyl diglyceryl tris(trimethylsiloxy)silylethyl dimethicone, and bis(polyglyceryl-3-oxyphenylpropyl) dimethicone.

Examples of commercially available products of the polyglycerol-modified silicone include "KF-6100", "KF-6104", "KF6106", and "KF-6105" manufactured by Shin-Etsu Chemical Co., Ltd., "DOWSI ES-5600 Silicone Glycerol Emulsifier" manufactured by Dow Toray Co., Ltd., and "SOFCARE GS-G" manufactured by Kao Corporation.

### [Amino-Modified Silicone]

Examples of the amino-modified silicone include silicones having an amino group or an ammonium group-containing group in a main chain or a side chain and not having a polyether structure, such as dimethylpolysiloxane (dimethicone) and methylphenylpolysiloxane, and preferable examples thereof include silicones represented by the following General Formula (2). 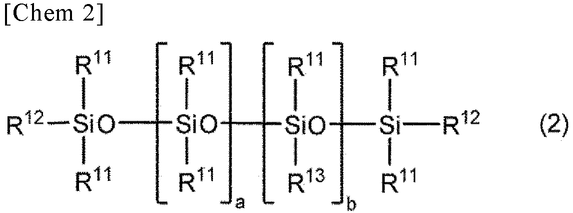 wherein R¹¹ each independently represents a methyl group or a phenyl group, and R¹² each independently represents an alkyl group having 1 or more and 30 or less carbons, a hydroxy group, or R¹³, R¹³ represents a monovalent group represented by -R¹⁴-Z¹ (R¹⁴ represents a single bond or a divalent hydrocarbon group having 1 or more and 20 or less carbons, and Z¹ represents a primary to tertiary amino group-containing group or an ammonium group-containing group), a represents the number of 0 or more and 3000 or less, b represents the number of 1 or more and 3000 or less, and a + b is the number of 100 or more and 20000 or less.

In General Formula (2), R¹¹ is preferably a methyl group, and R¹² is preferably a methyl group or R¹³.

R¹³ is a monovalent group represented by -R¹⁴-Z¹, and R¹⁴ is preferably a divalent hydrocarbon group having 1 or more and 20 or less carbons, more preferably an alkylene group having 1 or more and 20 or less carbons, even more preferably a linear or branched alkylene group having 1 or more and 6 or less carbons, even more preferably a methylene group, an ethylene group, a trimethylene group, a propylene group, a tetramethylene group, or a hexamethylene group, even more preferably a trimethylene group or a propylene group.

Z¹ is a primary to tertiary amino group-containing group or an ammonium group-containing group, represented by -N(R¹⁵)₂, - NR¹⁵(CH₂)_{c}N(R¹⁶)₂ or -NR¹⁵(CH₂)_{c}N(R¹⁶)CO-R¹⁷ or ammonium group-containing group represented by -N⁺(R¹⁵)₃A⁻ or -NR¹⁵(CH₂) _{c}N+(R¹⁶ )₃A⁻. Here, R¹⁵ and R¹⁶ each independently represent a hydrogen atom or an alkyl group having 1 or more and 3 or less carbons, and are preferably a hydrogen atom or a methyl group. R¹⁷ represents an alkyl group having 1 or more and 3 or less carbons. A is a counter anion and represents Cl, Br or I. c represents the number of 1 or more and 6 or less, and preferably the number of 2 or more and 4 or less.

In General Formula (2), R¹³ is preferably -(CH₂)₃-NH₂, -(CH₂)₃-N(CH₃)₂, -(CH₂)₃-NH-(CH₂)₂-NH₂, or -(CH₂)₂-NH-(CH₂)₂-N(CH₃)₂, more preferably -(CH₂)₃-NH₂ or -(CH₂)₃-NH-(CH₂)₂-NH₂.

Preferable examples of the silicone represented by General Formula (2) include one or more selected from the group consisting of aminoethylaminopropyldimethicone [amodimethicone], aminopropyldimethicone, bis(aminopropyl)dimethicone, and bis(cetearyl)amodimethicone.

Examples of a commercially available product of the amino-modified silicone include "Silicone SF 8457 C" manufactured by Dow Toray Co., Ltd. "DOWSIL CF 1046" manufactured by Dow Toray Co., Ltd., which is a mixture of amino-modified silicone and dimethylpolysiloxane (D) described below, and the like may also be used.

One or two or more of the component (C) can be used. Among them, an amino-modified silicone is preferable from the viewpoint of further improving the texture improving effect of the object to be treated and the durability of the effect, in particular, from the viewpoint of the effect of suppressing the entanglement of hair or fibers for head decoration products when used for treating hair or fibers for head decoration products and the improvement of the durability thereof.

### (Component (D): Dimethylpolysiloxane)

The component (D) is a dimethylpolysiloxane. It is considered that containing the component (C) and the component (D) in the composition of the present invention improves the texture improving effect of the object to be treated and the durability of the effect, in particular, the effect of suppressing the entanglement of hair or fibers for head decoration products when used for treating hair or fibers for head decoration products and the durability thereof, and the moisture resistance.

The dimethylpolysiloxane used as the component (D) preferably has low volatility, and from this viewpoint, the viscosity at 25°C is preferably 500 mm²/s or more, more preferably 700 mm²/s or more, and even more preferably 1000 mm²/s or more.

It is more preferable that the component (D) contains (D1) a dimethylpolysiloxane having a viscosity at 25°C of 1000 mm²/s or more and 10000 mm²/s or less and (D2) a dimethylpolysiloxane having a viscosity at 25°C of 300000 mm²/s or more and 5000000 mm²/s or less from the viewpoint of the improvement of the texture improving effect of the object to be treated and the durability of the effect, in particular, the effect of suppressing entanglement of hair or fibers for head decoration products when used for treating hair or fibers for head decoration products and the durability of the effect, and the moisture resistance.

The viscosity of the component (D) is a value measured at 25°C in accordance with "Method for measuring viscosity of liquid" specified in JIS Z8803:2011 or ASTM D 445-46 T, and can be measured using, for example, an Ubbelohde viscometer.

When the component (D) contains the component (D1) and the component (D2), the mass ratio of the component (D1) to the component (D2) [(D1)/(D2)] is preferably 0.5 or more, more preferably 1.0 or more, even more preferably 2.0 or more, and preferably 20 or less, more preferably 10 or less, even more preferably 5.0 or less from the viewpoint of the improvement of the texture improving effect on the object to be treated and the durability of the effect, in particular, the effect of suppressing entanglement of hair or fibers for head decoration products when used for treating hair or fibers for head decoration products and the durability thereof, and the moisture resistance. The mass ratio [(D1)/(D2)] is preferably 0.5 or more and 20 or less, more preferably 1.0 or more and 10 or less, and even more preferably 2.0 or more and 5.0 or less.

When the component (D) contains the component (D1) and the component (D2), the total content of the component (D1) and the component (D2) in the component (D) is preferably 50 mass% or more, more preferably 60 mass% or more, even more preferably 70 mass% or more, even more preferably 80 mass% or more, even more preferably 90 mass% or more, and is 100 mass% or less from the viewpoint of further improving the texture improving effect of the object to be treated and the durability of the effect, in particular, from the viewpoint of the effect of suppressing entanglement of hair or fibers for head decoration products when used for treating hair or fibers for head decoration products and the improvement of the durability thereof, and the improvement of the moisture resistance.

### (Component (E): Cationic Surfactant)

The composition of the present invention contains the component (E), a cationic surfactant, from the viewpoint of further improving the texture of the object to be treated.

Examples of the cationic surfactant include (i) an alkyltrimethylammonium salt, (ii) an alkoxyalkyltrimethylammonium salt, (iii) a dialkyldimethylammonium salt, (iv) an alkylamidoalkyltrimethylammonium salt, (v) alkyldimethylamine and a salt thereof, (vi) alkoxyalkyldimethylamine and a salt thereof, and (vii) alkylamidoalkyldimethylamine and a salt thereof.

Examples of (i) an alkyltrimethylammonium salt include an alkyltrimethylammonium salt having an alkyl group having preferably 12 or more and 24 or less carbons and more preferably 16 or more and 24 or less carbons, and specific examples thereof include cetyltrimethylammonium chloride (cetrimonium chloride), stearyltrimethylammonium chloride (steartrimonium chloride), and behenyltrimethylammonium chloride (behentrimonium chloride).

Examples of (ii) an alkoxyalkyltrimethylammonium salt include an alkoxyalkyltrimethylammonium salt having an alkoxy group having preferably 12 or more and 22 or less carbons and more preferably 16 or more and 20 or less carbons, and specific examples thereof include stearoxypropyltrimethylammonium chloride, stearoxyethyltrimethylammonium chloride, and stearoxyhydroxypropyltrimethylammonium chloride.

Examples of (iii) a dialkyldimethylammonium salt include a dialkyldimethylammonium salt having an alkyl group having preferably 12 or more and 22 or less carbons and more preferably 16 or more and 20 or less carbons, and specific examples thereof include distearyldimethylammonium chloride.

Examples of (iv) an alkylamidoalkyltrimethylammonium salt include an alkylamidoalkyltrimethylammonium salt having an alkyl group having preferably **11** or more and 21 or less carbons and more preferably 13 or more and 19 or less carbons, and specific examples thereof include palmitamidopropyltrimethylammonium chloride (palmitamidopropyltrimonium chloride).

(v) Alkyldimethylamine, (vi) alkoxyalkyldimethylamine, and (vii) alkylamidoalkyldimethylamine react with an acid to form a tertiary amine salt, and serve as a cationic surfactant.

The alkyl group in (v) alkyldimethylamine and a salt thereof and the alkoxy group in (vi) alkoxyalkyldimethylamine and a salt thereof are preferably alkyl groups having 12 or more and 22 or less carbons, and more preferably 16 or more and 20 or less carbons.

The alkyl group of the alkylamide moiety in (vii) alkylamidoalkyldimethylamine and a salt thereof is preferably an alkyl group having 11 or more and 21 or less carbons, more preferably 15 or more and 19 or less carbons.

The amines of (v) to (vii) may be caused to react with an acid in advance and blended to the composition as a salt, or may be added to the composition as it is as an amine and further added an acid to the composition to form a salt in the composition. Thus, herein, the amine and the salt thereof are defined as a cationic surfactant. The content or blending amount thereof is converted in terms of the mass of the amine.

Examples of the salts of the amines of (v) to (vii) include a salt with an organic acid or an inorganic acid. Examples of the organic acid include monocarboxylic acids such as acetic acid and propionic acid; dicarboxylic acids such as malonic acid, succinic acid, glutaric acid, adipic acid, maleic acid, fumaric acid, and phthalic acid; polycarboxylic acids such as polyglutamic acid; hydroxycarboxylic acids such as glycolic acid, lactic acid, hydroxyacrylic acid, glyceric acid, malic acid, tartaric acid, and citric acid; and acidic amino acids such as glutamic acid and aspartic acid. Examples of the inorganic acid include hydrochloric acid, sulfuric acid, and phosphoric acid. Among these, organic acids are preferable, and one or more selected from the group consisting of dicarboxylic acids, hydroxycarboxylic acids, and acidic amino acids are more preferable. The dicarboxylic acid is more preferably at least one selected from the group consisting of maleic acid and succinic acid. The hydroxycarboxylic acid is more preferably one or more selected from the group consisting of glycolic acid, lactic acid, and malic acid. Glutamic acid is more preferable as the acidic amino acid.

Examples of (v) alkyldimethylamine and a salt thereof include N,N-dimethylbehenylamine, N,N-dimethylstearylamine, and organic acid salts thereof, and a lactate salt of N,N-dimethylbehenylamine, a glycolic acid salt of N,N-dimethylstearylamine, and the like are preferable.

Examples of (vi) alkoxyalkyldimethylamine and a salt thereof include N,N-dimethyl-3-hexadecyloxypropylamine, N,N-dimethyl-3-octadecyloxypropylamine, and an organic acid salt thereof, and N,N-dimethyl-3-hexadecyloxypropylamine or a lactate salt thereof, and N,N-dimethyl-3-octadecyloxypropylamine(stearoxypropyldimethylamine) or a lactate salt thereof are preferable.

Examples of (vii) alkylamidoalkyldimethylamine and a salt thereof include N-[3-(dimethylamino)propyl]docosanamide, N-[3-(dimethylamino)propyl]stearamide, and organic acid salts thereof, and lactate salt of N-[3-(dimethylamino)propyl]docosanamide and glycolic acid salt of N-[3-(dimethylamino)propyl]stearamide are preferable.

One or two or more of the component (E) can be used.

Among these, from the viewpoint of further improving the texture of the object to be treated, the component (E) is preferably one or more selected from the group consisting of (i) an alkyltrimethylammonium salt, (ii) an alkoxyalkyltrimethylammonium salt, (iii) a dialkyldimethylammonium salt, (iv) an alkylamidoalkyltrimethylammonium salt, (v) alkyldimethylamine and a salt thereof, (vi) alkoxyalkyldimethylamine and a salt thereof, and (vii) alkylamidoalkyldimethylamine and a salt thereof, more preferably one or more selected from the group consisting of (i) an alkyltrimethylammonium salt and (vi) alkoxyalkyldimethylamine and a salt thereof, and even more preferably one or more selected from the group consisting of cetyltrimethylammonium chloride, stearyltrimethylammonium chloride, behenyltrimethylammonium chloride, N,N-dimethyl-3-hexadecyloxypropylamine or a lactate salt thereof, and N,N-dimethyl-3-octadecyloxypropylamine-(stearoxypropyldimethylamine) or a lactate salt thereof, even more preferably one or more selected from the group consisting of behenyltrimethylammonium chloride, and N,N-dimethyl-3-octadecyloxypropylamine(stearoxypropyldimethylamine) or a lactate salt thereof.

### (Component (F): Higher Alcohol)

The composition of the present invention contains (F) a higher alcohol from the viewpoint of further improving the texture of the object to be treated.

Examples of the higher alcohol include aliphatic monohydric alcohols having 12 or more carbons, preferably 12 or more and 22 or less carbons. Examples of the higher alcohol include one or more selected from the group consisting of lauryl alcohol, cetyl alcohol, myristyl alcohol, oleyl alcohol, stearyl alcohol, isostearyl alcohol, 2-octyldodecanol, behenyl alcohol, and cetostearyl alcohol.

Among the above, the component (F) is preferably one or more selected from the group consisting of cetyl alcohol, myristyl alcohol, stearyl alcohol, and behenyl alcohol from the viewpoint of further improving the texture of the object to be treated.

### (Component (G): Nonionic Polymer)

The composition of the present invention preferably further contains (G) a nonionic polymer from the viewpoint of further improving the texture improving effect of the object to be treated and the durability of the effect, in particular, from the viewpoint of improving the effect of suppressing entanglement of hair or fibers for head decoration products when used for treating hair or fibers for head decoration products and the durability thereof, and from the viewpoint of improving the moisture resistance. It is considered that when the component (G) is used, the rigid crosslinked structure of the polyion complex formed by the component (A) and the component (B) is reinforced by complexing with the long-chain and flexible non-crosslinked structure of the component (G). Since a hydrogen bond can be formed between the component (B) and the component (G), it is considered that the component (G) is held in the crosslinked structure by the component (B) to form a network having higher strength in the film. It is considered that, as a result, the effect of improving the texture of the object to be treated, the durability of the effect, and the moisture resistance are further improved.

In the present specification, the "nonionic polymer" means a polymer having substantially no cationic group, anionic group, or amphoteric group, which are ionic groups.

Examples of the component (G) include polyalkylene glycol or a derivative thereof, a polymer containing a constituent unit derived from vinylpyrrolidone, a polymer containing a constituent unit derived from a (meth)acrylic acid ester, polyvinyl alcohol, polyacrylamide, and polycaprolactam, and one or two or more thereof can be used.

Examples of the polyalkylene glycol include homopolymers and copolymers of alkylene glycols in which the number of carbons of alkylene is 2 or more and 6 or less, preferably 2 or more and 4 or less, and more preferably 2 or more and 3 or less. Specific examples thereof include polyethylene glycol, polypropylene glycol, polytrimethylene ether glycol, polytetramethylene ether glycol, a polyethylene glycol-polypropylene glycol copolymer (polyoxyethylene polyoxypropylene glycol), a polyethylene glycol-polytrimethylene ether glycol copolymer, a polyethylene glycol-polytetramethylene ether glycol copolymer, and a polypropylene glycol-polytetramethylene ether glycol copolymer. Among these, one or more selected from the group consisting of polyethylene glycol, polypropylene glycol, and a polyethylene glycol-polypropylene glycol copolymer is preferable, and one or more selected from the group consisting of polyethylene glycol and a polyethylene glycol-polypropylene glycol copolymer is more preferable, from the viewpoints of the effect of improving the texture of the object to be treated and the improvement of the durability of the effect, in particular, the effect of suppressing entanglement of hair or fibers for head decoration products when used for treating hair or fibers for head decoration products and the improvement of the durability thereof, from the viewpoint of the improvement of moisture resistance, and from the viewpoint of availability.

Examples of the polyalkylene glycol derivative include terminal-modified products of the above-described polyalkylene glycols, such as polyalkylene glycol monoalkyl ethers, polyalkylene glycol dialkyl ethers, polyalkylene glycol monoaryl ethers, polyalkylene glycol diaryl ethers, polyalkylene glycol monofatty acid esters, polyalkylene glycol difatty acid esters, and polyalkylene glycol diglycidyl ethers. Among these, one or more selected from the group consisting of a polyalkylene glycol monoalkyl ether, a polyalkylene glycol dialkyl ether, a polyalkylene glycol monofatty acid ester, and a polyalkylene glycol difatty acid ester is preferable, and one or more selected from the group consisting of a polyalkylene glycol monofatty acid ester and a polyalkylene glycol difatty acid ester is more preferable, from the viewpoint of the texture improving effect of the object to be treated and the improvement of the durability of the effect, in particular, the effect of suppressing entanglement of hair or fibers for head decoration products when used for treating hair or fibers for head decoration products and the improvement of the durability thereof, and from the viewpoint of availability.

The number of carbons of the alkyl group in the mono- or dialkyl ether of polyalkylene glycol and the number of carbons of the fatty acid in the mono- or di-fatty acid ester of polyalkylene glycol are preferably 1 or more and 36 or less, more preferably 4 or more and 32 or less, even more preferably 8 or more and 24 or less, and even more preferably 12 or more and 22 or less. The number of carbons of the aryl group in the mono- or diaryl ether of polyalkylene glycol is preferably 6 or more and 36 or less, more preferably 6 or more and 32 or less, even more preferably 6 or more and 24 or less, and even more preferably 6 or more and 22 or less.

Specific examples of the polyalkylene glycol derivative include polyethylene glycol monomethyl ether, polyethylene glycol dimethyl ether, polyethylene glycol monobutyl ether, polyethylene glycol dibutyl ether, polyethylene glycol monooctyl ether, polyethylene glycol monodecyl ether, polyethylene glycol monododecyl ether, polyethylene glycol monotridecyl ether, polyethylene glycol-bisphenol A ether, polyethylene glycol dilaurate, polyethylene glycol distearate, polyethylene glycol diglycidyl ether, polyethylene glycol-polypropylene glycol-monomethyl ether, polyethylene glycol-polypropylene glycol-dimethyl ether, polyethylene glycol-polypropylene glycol-monobutyl ether, polyethylene glycol-polypropylene glycol-dibutyl ether, polyethylene glycol-polypropylene glycol-monooctyl ether, polyethylene glycol-polypropylene glycol-monodecyl ether, polyethylene glycol-polypropylene glycol-monododecyl ether, polyethylene glycol-polypropylene glycol-monotridecyl ether, polyethylene glycol-polypropylene glycol-bisphenol A ether, polyethylene glycol-polypropylene glycol-dilaurate, polyethylene glycol distearate-polypropylene glycol, and polyethylene glycol-polypropylene glycol-diglycidyl ether, and one or two or more of them can be used.

Examples of the polymer containing a constituent unit derived from vinylpyrrolidone include a homopolymer of vinylpyrrolidone and a copolymer of vinylpyrrolidone and a nonionic monomer. Specific examples thereof include polyvinylpyrrolidone, a vinylpyrrolidone/vinyl acetate copolymer, and a vinylpyrrolidone/methacrylamide/vinylimidazole copolymer.

Examples of the polymer containing a constituent unit derived from a (meth)acrylic acid ester include a homopolymer of a (meth)acrylic acid ester, and a copolymer of a (meth)acrylic acid ester and a nonionic monomer such as (meth)acrylamide, dimethylacrylamide, vinyl acetate, or vinyl methyl ether. Examples of the (meth)acrylic acid ester include (meth)acrylic acid hydroxyalkyl ester and (meth)acrylic acid alkyl ether in addition to the (meth)acrylic acid alkyl ester described above.

Specific examples of the polymer containing a constituent unit derived from a (meth)acrylic acid ester include a (dimethylacrylamide/hydroxyethyl acrylate/methoxyethyl acrylate) copolymer and a (hydroxyethyl acrylate/methoxyethyl acrylate) copolymer.

Among the above, the component (G) is preferably one or more selected from the group consisting of polyalkylene glycol or a derivative thereof and a polymer containing a constituent unit derived from vinylpyrrolidone, more preferably one or more selected from the group consisting of polyethylene glycol, polypropylene glycol, a polyethylene glycol-polypropylene glycol copolymer, a mono- or di-fatty acid ester thereof, and polyvinylpyrrolidone, and even more preferably one or more selected from the group consisting of polyethylene glycol, a polyethylene glycol-polypropylene glycol copolymer, a mono- or di-fatty acid ester thereof, and polyvinylpyrrolidone, from the viewpoint of the texture improving effect of the object to be treated and the improvement of the durability of the effect, in particular, the effect of suppressing entanglement of hair or fibers for head decoration products when used for treating hair or fibers for head decoration products and the improvement of the durability thereof, from the viewpoint of the improvement of the moisture resistance, and from the viewpoint of the availability.

The weight average molecular weight (Mw) of the component (G) is preferably 2000 or more, more preferably 5000 or more, even more preferably 10000 or more, and preferably 2000000 or less, more preferably 1500000 or less from the viewpoint of the texture improving effect of the object to be treated and the improvement of the durability of the effect, in particular, the effect of suppressing entanglement of hair or fibers for head decoration products when used for treating hair or fibers for head decoration products and the improvement of the durability thereof, from the viewpoint of the improvement of the moisture resistance, and from the viewpoint of the availability. The weight average molecular weight of the component (G) is preferably 2000 or more and 2000000 or less, more preferably 5000 or more and 1500000 or less, and even more preferably 10000 or more and 1500000 or less.

The weight average molecular weight of the component (G) can be measured by gel permeation chromatography (GPC).

### (Component (H): Acid)

From the viewpoint of stably forming a polyion complex, the composition of the present invention preferably further contains (H) an acid.

As the component (H), an organic acid or an inorganic acid can be used, and from the viewpoint of use in a cosmetic composition or a fiber treatment composition for head decoration products, an organic acid is preferable. Examples of the organic acid include a carboxylic acid-based compound and a sulfonic acid-based compound other than the component (B), and the organic acid is preferably a compound having a molecular weight of 500 or less, more preferably 200 or less.

Examples of the carboxylic acid-based compound include aliphatic monocarboxylic acids having 4 or less carbons, such as acetic acid, propionic acid, and butanoic acid; aromatic monocarboxylic acids such as benzoic acid; aliphatic dicarboxylic acids such as malonic acid, succinic acid, glutaric acid, adipic acid, maleic acid, and fumaric acid; aromatic dicarboxylic acids such as phthalic acid and isophthalic acid; polycarboxylic acids such as polyglutamic acid; hydroxycarboxylic acids such as lactic acid, malic acid, glycolic acid, hydroxyacrylic acid, glyceric acid, tartaric acid, and citric acid; and acidic amino acids such as glutamic acid and aspartic acid, and one or two or more of these can be used.

Examples of the sulfonic acid-based compound include aliphatic sulfonic acids such as methanesulfonic acid and ethanesulfonic acid; aromatic sulfonic acids such as p-toluene sulfonic acid and naphthalene sulfonic acid; , and one or two or more of these can be used.

From the viewpoint of stably forming the polyion complex to form a highly stable composition and from the viewpoint of use in a cosmetic composition or a fiber treatment composition for head decoration products, the organic acid is preferably one or more selected from the group consisting of aliphatic dicarboxylic acid, hydroxycarboxylic acid, and aromatic sulfonic acid, more preferably one or more selected from the group consisting of succinic acid, lactic acid, malic acid, glycolic acid, p-toluenesulfonic acid, and naphthalenesulfonic acid, even more preferably one or more selected from the group consisting of succinic acid and lactic acid, and even more preferably lactic acid.

At least a part of the organic acid may be in the form of an organic acid salt at the time of blending. The salt of the organic acid is preferably an alkali metal salt or an alkali metal salt of an organic acid, more preferably an alkali metal salt, even more preferably one or more selected from the group consisting of a sodium salt and a potassium salt, and even more preferably a sodium salt, from the viewpoint of use in a cosmetic composition or a fiber treatment composition for head decoration products and from the viewpoint of availability.

The proportion of the organic acid salt in the total amount of the organic acid and the organic acid salt is preferably 50 mass% or less, more preferably 20 mass% or less, even more preferably 5 mass% or less, and may be 0 mass%, from the viewpoint of ease of forming the polyion complex and improvement in stability of the composition. The proportion (mass%) of the organic acid salt referred to herein means mass% in terms of organic acid.

### (Water)

The composition of the present invention preferably contains water from the viewpoint of forming an emulsion composition. The water used in the composition of the present invention is preferably deionized water or distilled water. Tap water, ground water or the like sterilized with hypochlorous acid or the like may be used as long as the stability of the composition is not impaired.

### (Content)

The content or blending amount of each component in the composition of the present invention is preferably as follows from the viewpoint of the texture improving effect of the object to be treated and the improvement of the durability of the effect, in particular, the effect of suppressing entanglement of hair or fibers for head decoration products when used for treating hair or fibers for head decoration products and the improvement of the durability thereof, from the viewpoint of the improvement of moisture resistance, and from the viewpoint of the improvement of the stability of the composition.

The content of the component (A) in the composition is preferably 0.01 mass% or more, more preferably 0.02 mass% or more, even more preferably 0.05 mass% or more, even more preferably 0.1 mass% or more, and even more preferably 0.2 mass% or more from the viewpoint of ease of forming a polyion complex, improvement in texture improvement effect of the object to be treated and durability of the effect, particularly improvement in entanglement suppression effect of hair or fibers for head decoration products when used for treating hair or fibers for head decoration products and durability thereof, and improvement in moisture resistance. From the viewpoint of improving the stability of the composition, the content is preferably 3.0 mass% or less, more preferably 2.5 mass% or less, even more preferably 2.0 mass% or less, even more preferably 1.5 mass% or less, and even more preferably 1.0 mass% or less. The content of the component (A) in the composition is preferably 0.01 mass% or more and 3.0 mass% or less, more preferably 0.02 mass% or more and 2.5 mass% or less, even more preferably 0.05 mass% or more and 2.0 mass% or less, even more preferably 0.1 mass% or more and 1.5 mass% or less, even more preferably 0.2 mass% or more and 1.5 mass% or less, and even more preferably 0.2 mass% or more and 1.0 mass% or less.

The content of the component (B) in the composition is preferably 0.005 mass% or more, more preferably 0.01 mass% or more, even more preferably 0.02 mass% or more, and even more preferably 0.025 mass% or more, from the viewpoint of ease of forming a polyion complex, from the viewpoint of the effect of improving the texture of the object to be treated and the durability of the effect, particularly from the viewpoint of the effect of suppressing entanglement of hair or fibers for head decoration products when used for treating hair or fibers for head decoration products and the durability thereof, and from the viewpoint of improving moisture resistance. From the viewpoint of improving the stability of the composition, the content is preferably 3.0 mass% or less, more preferably 2.0 mass% or less, even more preferably 1.5 mass% or less, even more preferably 1.0 mass% or less, even more preferably 0.5 mass% or less, even more preferably 0.3 mass% or less, even more preferably 0.2 mass% or less, and even more preferably 0.1 mass% or less. The content of the component (B) in the composition is preferably 0.005 mass% or more and 3.0 mass% or less, more preferably 0.01 mass% or more and 2.0 mass% or less, even more preferably 0.02 mass% or more and 1.5 mass% or less, even more preferably 0.02 mass% or more and 1.0 mass% or less, even more preferably 0.02 mass% or more and 0.5 mass% or less, even more preferably 0.02 mass% or more and 0.3 mass% or less, even more preferably 0.02 mass% or more and 0.3 mass% or less, even more preferably 0.02 mass% or more and 0.2 mass% or less, even more preferably 0.02 mass% or more and 0.1 mass% or less, and even more preferably 0.025 mass% or more and 0.1 mass% or less.

The total content of the component (A) and the component (B) in the composition is preferably 0.02 mass% or more, more preferably 0.04 mass% or more, even more preferably 0.05 mass% or more, even more preferably 0.1 mass% or more, even more preferably 0.2 mass% or more, and even more preferably 0.3 mass% or more from the viewpoint of the texture improving effect of the object to be treated and the improvement of the durability of the effect, in particular, the effect of suppressing entanglement of hair or fibers for head decoration products when used for treating hair or fibers for head decoration products, the improvement of the durability of the effect, and the improvement of the moisture resistance. From the viewpoint of improving the stability of the composition, the content is preferably 5.0 mass% or less, more preferably 4.0 mass% or less, even more preferably 3.0 mass% or less, and even more preferably 2.0 mass% or less. The total content of the component (A) and the component (B) in the composition is preferably 0.02 mass% or more and 5.0 mass% or less, more preferably 0.04 mass% or more and 4.0 mass% or less, even more preferably 0.05 mass% or more and 3.0 mass% or less, even more preferably 0.1 mass% or more and 2.0 mass% or less, even more preferably 0.2 mass% or more and 2.0 mass% or less, and even more preferably 0.3 mass% or more and 2.0 mass% or less.

The ratio between the contents of the component (A) and the component (B) contained in the composition is such that the mass ratio of the component (A) to the component (B) [(A)/(B)] is 4.5 or more, preferably 4.7 or more, more preferably 4.8 or more, even more preferably 5.0 or more, even more preferably 5.5 or more, even more preferably 6.0 or more, even more preferably 6.2 or more, and even more preferably 6.5 or more, from the viewpoint of the ease of forming the polyion complex, from the viewpoints of the texture improving effect of the object to be treated and the improvement of the durability of the effect, in particular, the effect of suppressing entanglement of hair or fibers for head decoration products when used for treating hair or fibers for head decoration products and the improvement of the durability thereof, and from the viewpoint of the improvement of the moisture resistance. From the viewpoint of improving the stability of the composition, the ratio is preferably 20 or less, more preferably 18 or less, even more preferably 16 or less, and even more preferably 15 or less. The mass ratio [(A)/(B)] is 4.5 or more, preferably 4.5 or more and 20 or less, more preferably 4.7 or more and 18 or less, even more preferably 4.8 or more and 18 or less, even more preferably 5.0 or more and 18 or less, even more preferably 5.5 or more and 16 or less, even more preferably 6.0 or more and 16 or less, even more preferably 6.2 or more and 15 or less, and even more preferably 6.5 or more and 15 or less.

**In** the composition of the present invention, the mass ratio between the component (A) and the component (B) acts more dominantly than the cationic charge/anionic charge ratio of the component (A) and the component (B) for the effect of the present invention.

The ratio between the number of moles of cationic charges of the component (A) and the number of moles of anionic charges of the component (B) in the composition (cationic charges of the component (A)/anionic charges of the component (B)) is preferably 10/90 to 95/5, more preferably 20/80 to 95/5, even more preferably 25/75 to 90/10, even more preferably 30/70 to 90/10, even more preferably 40/60 to 90/10, even more preferably 50/50 to 90/10, and even more preferably 52/48 to 90/10 from the viewpoint of the texture improving effect of the object to be treated and the improvement of the durability of the effect, in particular, the effect of suppressing entanglement of hair or fibers for head decoration products when used for treating hair or fibers for head decoration products, the improvement of the durability of the effect, and the improvement of the moisture resistance.

The content of the component (C) in the composition is preferably 0.01 mass% or more, more preferably 0.02 mass% or more, even more preferably 0.05 mass% or more, and even more preferably 0.1 mass% or more, from the viewpoints of the texture improving effect of the object to be treated and the improvement of the durability of the effect, in particular, the improvement of the effect of suppressing entanglement of hair or fibers for head decoration products when used for treating hair or fibers for head decoration products and the improvement of the durability thereof, and from the viewpoint of the improvement of the moisture resistance. From the viewpoint of improving the stability of the composition, the content is preferably 5.0 mass% or less, more preferably 3.0 mass% or less, and even more preferably 2.0 mass% or less. The content of the component (C) in the composition is preferably 0.01 mass% or more and 5.0 mass% or less, more preferably 0.02 mass% or more and 3.0 mass% or less, even more preferably 0.05 mass% or more and 2.0 mass% or less, and even more preferably 0.1 mass% or more and 2.0 mass% or less.

The content of the component (D) in the composition is preferably 0.05 mass% or more, more preferably 0.1 mass% or more, even more preferably 0.3 mass% or more, and even more preferably 0.5 mass% or more, from the viewpoint of the effect of improving the texture of the object to be treated and the durability of the effect, in particular, the effect of suppressing entanglement of hair of fibers for head decoration products when used for treating hair or fibers for head decoration products and the durability thereof, and from the viewpoint of improving the moisture resistance. From the viewpoint of improving the stability of the composition, the content is preferably 15 mass% or less, more preferably 10 mass% or less, and even more preferably 8.0 mass% or less. The content of the component (D) in the composition is preferably 0.05 mass% or more and 15 mass% or less, more preferably 0.1 mass% or more and 10 mass% or less, even more preferably 0.3 mass% or more and 8.0 mass% or less, and even more preferably 0.5 mass% or more and 8.0 mass% or less.

The total content of the component (C) and the component (D) in the composition is preferably 0.06 mass% or more, more preferably 0.1 mass% or more, even more preferably 0.3 mass% or more, even more preferably 0.5 mass% or more, and even more preferably 0.6 mass% or more from the viewpoint of the texture improving effect of the object to be treated and the improvement of the durability of the effect, in particular, the effect of suppressing entanglement of hair or fibers for head decoration products when used for treating hair or fibers for head decoration products and the improvement of the durability thereof, and from the viewpoint of the improvement of the moisture resistance. From the viewpoint of improving the stability of the composition, the content is preferably 20 mass% or less, more preferably 15 mass% or less, and even more preferably 10 mass% or less. The total content of the component (C) and the component (D) in the composition is preferably 0.06 mass% or more and 20 mass% or less, more preferably 0.1 mass% or more and 15 mass% or less, even more preferably 0.3 mass% or more and 10 mass% or less, even more preferably 0.5 mass% or more and 10 mass% or less, and even more preferably 0.6 mass% or more and 10 mass% or less.

The ratio between the contents of the component (C) and the component (D) in the composition is such that the mass ratio of the component (C) to the component (D) [(C)/(D)] is preferably 0.02 or more, more preferably 0.05 or more, even more preferably 0.1 or more, and is preferably 20 or less, more preferably 10 or less, even more preferably 5.0 or less, even more preferably 2.0 or less, even more preferably 1.0 or less, even more preferably 0.5 or less, from the viewpoint of the ease of formation of the polyion complex, from the viewpoints of the texture improving effect of the object to be treated and the improvement of the durability of the effect, in particular, the effect of suppressing entanglement of hair or fibers for head decoration products when used for treating hair or fibers for head decoration products and the improvement of the durability thereof, and from the viewpoint of the improvement of the moisture resistance. The mass ratio [(C)/(D)] is preferably 0.02 or more and 20 or less, more preferably 0.02 or more and 10 or less, even more preferably 0.05 or more and 5.0 or less, even more preferably 0.05 or more and 2.0 or less, even more preferably 0.1 or more and 1.0 or less, and even more preferably 0.1 or more and 0.5 or less.

The content of the component (E) in the composition is preferably 0.1 mass% or more, more preferably 0.2 mass% or more, and even more preferably 0.5 mass% or more from the viewpoint of further improving the texture of the object to be treated. From the viewpoint of improving the stability of the composition, the content is preferably 10 mass% or less, more preferably 5.0 mass% or less, and even more preferably 3.5 mass% or less. The content of the component (E) in the composition is preferably 0.1 mass% or more and 10 mass% or less, more preferably 0.2 mass% or more and 5.0 mass% or less, and even more preferably 0.5 mass% or more and 3.5 mass% or less.

The content of the component (F) in the composition is preferably 0.1 mass% or more, more preferably 0.2 mass% or more, even more preferably 0.5 mass% or more, and even more preferably 1.0 mass% or more from the viewpoint of further improving the texture of the object to be treated. From the viewpoint of improving the stability of the composition, the content is preferably 15 mass% or less, more preferably 12 mass% or less, and even more preferably 10 mass% or less. The content of the component (F) in the composition is preferably 0.1 mass% or more and 15 mass% or less, more preferably 0.2 mass% or more and 12 mass% or less, even more preferably 0.5 mass% or more and 12 mass% or less, and even more preferably 1.0 mass% or more and 10 mass% or less.

The total content of the components (A) to (F) in the composition is preferably 1.0 mass% or more, more preferably 2.0 mass% or more, even more preferably 3.0 mass% or more, and even more preferably 5.0 mass% or more from the viewpoints of the texture improving effect of the object to be treated and the improvement of the durability of the effect, in particular, the effect of suppressing entanglement of hair of fibers for head decoration products when used for treating hair or fibers for head decoration products and the improvement of the durability thereof, from the viewpoint of the improvement of the moisture resistance, and from the viewpoint of the improvement of the stability of the composition. From the viewpoint of improving the stability of the composition, the total content is preferably 50 mass% or less, more preferably 40 mass% or less, even more preferably 30 mass% or less, more preferably 25 mass% or less, and even more preferably 21.5 mass% or less. The total content of the components (A) to (F) in the composition is preferably 1.0 mass% or more and 50 mass% or less, more preferably 2.0 mass% or more and 40 mass% or less, even more preferably 3.0 mass% or more and 30 mass% or less, even more preferably 5.0 mass% or more and 25 mass% or less, and even more preferably 5.0 mass% or more and 21.5 mass% or less.

When the composition contains (G) a nonionic polymer, the content of the component (G) in the composition is preferably 0.01 mass% or more, more preferably 0.02 mass% or more, even more preferably 0.05 mass% or more, and even more preferably 0.1 mass% or more from the viewpoint of the texture improving effect of the object to be treated and the improvement of the durability of the effect, in particular, the effect of suppressing entanglement of hair or fibers for head decoration products when used for treating hair or fibers for head decoration products, the improvement of the durability of the effect, and the improvement of the moisture resistance. From the viewpoint of improving the stability of the composition, the content is preferably 3.0 mass% or less, more preferably 2.5 mass% or less, even more preferably 2.0 mass% or less, even more preferably 1.5 mass% or less, and even more preferably 1.0 mass% or less. The content of the component (G) in the composition is preferably 0.01 mass% or more and 3.0 mass% or less, more preferably 0.02 mass% or more and 2.5 mass% or less, even more preferably 0.05 mass% or more and 2.0 mass% or less, even more preferably 0.1 mass% or more and 1.5 mass% or less, and even more preferably 0.1 mass% or more and 1.0 mass% or less.

When the composition contains (G) a nonionic polymer, the mass ratio of the component (G) to the total amount of the component (A) and the component (B) [(G)/{(A) + (B)}] in the composition is preferably 0.2 or more, more preferably 0.3 or more, even more preferably 0.4 or more, even more preferably 0.5 or more, even more preferably 0.6 or more, even more preferably 0.8 or more, and preferably 20 or less, more preferably 15 or less, even more preferably 10 or less, even more preferably 8.0 or less, even more preferably 5.0 or less, even more preferably 2.0 or less, even more preferably 1.5 or less from the viewpoint of the texture improving effect of the object to be treated and the improvement of the durability of the effect, in particular, the effect of suppressing entanglement of hair or fibers for head decoration products when used for treating hair or fibers for head decoration products and the improvement of the durability of the effect, and from the viewpoint of improving the moisture resistance. The mass ratio [(G)/{(A) + (B)}] is preferably 0.2 or more and 20 or less, more preferably 0.3 or more and 15 or less, even more preferably 0.4 or more and 10 or less, even more preferably 0.5 or more and 8.0 or less, even more preferably 0.6 or more and 5.0 or less, even more preferably 0.8 or more and 2.0 or less, and even more preferably 0.8 or more and 1.5 or less.

When the composition contains (H) an acid, the content of the component (H) in the composition is preferably 0.01 mass% or more, more preferably 0.02 mass% or more, even more preferably 0.03 mass% or more, even more preferably 0.05 mass% or more, even more preferably 0.1 mass% or more, and even more preferably 0.2 mass% or more from the viewpoint of ease of forming a polyion complex. The content is preferably 5.0 mass% or less, more preferably 3.0 mass% or less, and even more preferably 2.5 mass% or less from the viewpoint of facilitating formation of a polyion complex and improving the stability of the composition. The content of the component (H) in the composition is preferably 0.01 mass% or more and 5.0 mass% or less, more preferably 0.02 mass% or more and 3.0 mass% or less, even more preferably 0.03 mass% or more and 3.0 mass% or less, even more preferably 0.05 mass% or more and 3.0 mass% or less, even more preferably 0.05 mass% or more and 3.0 mass% or less, even more preferably 0.1 mass% or more and 3.0 mass% or less, even more preferably 0.1 mass% or more and 2.5 mass% or less, and even more preferably 0.2 mass% or more and 2.5 mass% or less.

When the organic acid is blended in the form of an organic acid salt, the content is an amount converted to an organic acid.

However, when the composition contains any of (v) alkyldimethylamine and a salt thereof, (vi) alkoxyalkyldimethylamine and a salt thereof, and (vii) alkylamidoalkyldimethylamine and a salt thereof as the component (E), the content of the component (H) in the composition may be more than 5.0 mass%. This is because the presence of the component (H) as a counter anion of the amine is expected to improve the stability of the composition.

When the composition contains water, the content of water in the composition is preferably 10 mass% or more, more preferably 20 mass%% or more, and even more preferably 30 mass% or more from the viewpoint of ease of forming a polyion complex and from the viewpoint of improving the stability of the composition. The content is preferably 99 mass% or less, more preferably 95 mass% or less, and even more preferably 90 mass% or less from the viewpoint of improving the stability of the composition and the degree of freedom of blending. The content of water in the composition is preferably 10 mass% or more and 99 mass% or less, more preferably 20 mass% or more and 95 mass% or less, and even more preferably 30 mass% or more and 90 mass% or less. The content of water in the composition may be the balance of the components (A) to (F).

### (Other Components)

The composition of the present invention can contain, as optional components, an anionic surfactant, a nonionic surfactant, an amphoteric surfactant, an aromatic ring-containing alcohol such as benzyl alcohol or phenoxyethanol, a non-aromatic polyol such as dipropylene glycol, an oil agent other than the components (C), (D), and (F), an antioxidant, an anti-dandruff agent, a vitamin agent, a disinfectant, an anti-inflammatory agent, an antiseptic agent, a chelating agent, a moisturizing agent, a pearl agent, ceramides, a fragrance, a plant extract, an ultraviolet absorber, a pH adjusting agent, and the like.

### (pH)

From the viewpoint of safety, the pH of the composition is preferably 3.0 or more, more preferably 3.2 or more as the pH at 25°C of an aqueous solution obtained by diluting the composition with water by 20 times. From the viewpoint of improving the stability of the composition, the pH is preferably 7.5 or less, more preferably 7.0 or less, even more preferably 6.0 or less, and even more preferably 5.0 or less.

The pH can be measured by the method described in Examples using a pH meter.

### (Method for Producing Composition)

The method for producing the composition is not particularly limited, but when the composition is an emulsified composition, the composition is preferably produced by a method including the following steps 1 to 3, any one of steps 4-1 and 4-2, and step 5 in this order from the viewpoint of being able to produce a composition having a high texture improving effect of the object to be treated and durability of the effect, in particular, the effect of suppressing entanglement of hair or fibers for head decoration products when used for treating hair or fibers for head decoration products and durability of the effect, having excellent moisture resistance improving effect, and having further high stability.
Step 1: Step of preparing a solution 1 containing (B) an anionic polymer
Step 2: Step of mixing the solution 1 with (H) an acid or an aqueous solution thereof to prepare a mixture 2 containing (B) an anionic polymer, (H) an acid, and water
Step 3: Step of mixing the mixture 2 with (A) a cationic polymer to prepare a mixture 3 containing (A) a cationic polymer, (B) an anionic polymer, (H) an acid, and water
Step 4-1: Step of mixing (I) an aqueous phase containing water with (I) an oily phase containing (D) a cationic surfactant and (E) a higher alcohol to prepare (Ia) an emulsion, and then mixing (Ia) an emulsion with the mixture 3 to prepare an emulsion 4-1
Step 4-2: Step of mixing the mixture 3 with (I) an aqueous phase containing water to prepare (Ia) an aqueous phase, and then mixing (Ia) an aqueous phase with (I) an oily phase containing (D) a cationic surfactant and (E) a higher alcohol to prepare an emulsion 4-2
Step 5: Step of mixing the emulsion 4-1 or the emulsion 4-2 with (II) an oily phase containing (C) one or more modified silicones selected from the group consisting of a polyglycerol-modified silicone and an amino-modified silicone and (D) a dimethylpolysiloxane

According to the above method, the polyion complex can be efficiently formed without being inhibited by other components. As a result, it is considered that it is possible to produce a composition having a high texture improving effect on the object to be treated and durability of the effect, in particular, an effect of suppressing entanglement of hair or fibers for head decoration products when used for treating hair or fibers for head decoration products and the durability of the effect, having excellent moisture resistance improving effect, and further having high stability.

In the present specification, "aqueous phase" means a phase constituting an aqueous phase in the production process of an emulsion composition, and "oily phase" means a phase constituting an oil phase in the production process of an emulsion composition.

In the step 1, the solution 1 containing (B) an anionic polymer can be prepared by dissolving or dispersing (B) an anionic polymer in water. When a solution in which the component (B) is dissolved or dispersed in water is prepared, the component (B) may be dispersed in advance in an aqueous medium other than water, such as dipropylene glycol, and then dissolved or dispersed in water.

As a solution obtained by dissolving or dispersing the component (B) in water, a commercially available aqueous solution or aqueous dispersion can also be used. For example, a reaction liquid when the component (B) is produced using water as a reaction solvent may be used as a solution in which the component (B) is dissolved or dispersed in water.

In the step 3, (A) a cationic polymer may be mixed with the mixture 2 as it is, but from the viewpoint of ease of production, it is preferable to mix a solution obtained by dissolving or dispersing the component (A) in water with the mixture 2.

As the solution obtained by dissolving or dispersing the component (A) in water, a commercially available aqueous solution or aqueous dispersion can also be used. In addition, for example, a reaction liquid when the component (A) is produced using water as a reaction solvent may be used as a solution in which the component (A) is dissolved or dispersed in water.

When (G) a nonionic polymer is used, it is preferable that in the step 1, a solution obtained by dissolving or dispersing (B) an anionic polymer in water is mixed with (G) a nonionic polymer to prepare the solution 1 containing (B) an anionic polymer and (G) a nonionic polymer, and the solution 1 is subjected to the step 2 and subsequent steps.

With respect to the above-described embodiments, the present invention further discloses the following aspects.
<1> A cosmetic composition or a fiber treatment composition for head decoration products containing (A) a cationic polymer having a viscosity when in a 1 mass% aqueous solution form at 30°C of 1000 mPa·s or more, (B) an anionic polymer, (C) one or more modified silicones selected from the group consisting of a polyglycerol-modified silicone and an amino-modified silicone, (D) a dimethylpolysiloxane, (E) a cationic surfactant, and (F) a higher alcohol, wherein a mass ratio [(A)/(B)] of the component (A) to the component (B) is 4.5 or more.
<2> The composition according to <1>, wherein the viscosity of a 1 mass% aqueous solution of the component (A) at 30°C is 1000 mPa·s or more, preferably 1000 mPa·s or more and 100000 mPa·s or less, more preferably 1300 mPa·s or more and 80000 mPa·s or less, even more preferably 1500 mPa·s or more and 50000 mPa·s or less, even more preferably 1700 mPa·s or more and 30000 mPa·s or less, and even more preferably 1800 mPa·s or more and 25000 mPa·s or less.
<3> The composition according to <1> or <2>, wherein the component (A) has a cationic charge density of preferably 0.1 mmol/g or more and 20 mmol/g or less, more preferably 0.2 mmol/g or more and 15 mmol/g or less, 0.2 mmol/g or more and 12 mmol/g or less, even more preferably 0.3 mmol/g or more and 10 mmol/g or less, even more preferably 0.3 mmol/g or more and 7.0 mmol/g or less, and even more preferably 0.5 mmol/g or more and 5.0 mmol/g or less.
<4> The composition according to any one of <1> to <3>, wherein the component (A) is one or more selected from the group consisting of a cationized polyvinyl alcohol, a polyethyleneimine, a methacryloylethyltrimethylammonium salt polymer, a quaternized dialkylaminoalkyl (meth)acrylate polymer, a diallyl quaternized ammonium salt polymer, a methacrylamidopropyltrimethylammonium salt polymer, a vinylimidazolium trichloride-vinylpyrrolidone copolymer (polyquaternium-16), a vinylpyrrolidone-alkylamino (meth)acrylate copolymer, a vinylpyrrolidone-alkylamino (meth)acrylate-vinylcaprolactam copolymer, an alkylacrylamide-(meth)acrylate-alkylaminoalkylacrylamide-polyethylene glycol (meth)acrylate copolymer, and an adipic acid-dimethylaminohydroxypropylethylenetriamine copolymer.
<5> The composition according to any one of <1> to <4>, wherein the component (A) is a polymer including a constituent unit represented by General Formula (1) described below: 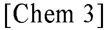 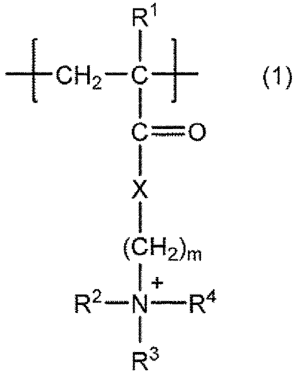 wherein R¹ is a hydrogen atom or a methyl group, and R² to R⁴ are each independently an alkyl group having 1 or more and 4 or less carbons, X is -O- or -NH-, and m is the number of 1 to 4.
<6> The composition according to <6>, wherein a content of the constituent unit represented by General Formula (1) in the component (A) is preferably 8.0 mol% or more, more preferably 10 mol% or more, and is 100 mol% or less, based on all constituent units constituting the component (A).
<7> The composition according to any one of <1> to <6>, wherein the component (A) is preferably one or more selected from the group consisting of a methacryloylethyltrimethylammonium salt polymer and a quaternized dialkylaminoalkyl (meth)acrylate salt polymer, more preferably one or more selected from the group consisting of a methacryloylethyltrimethylammonium chloride polymer (polyquaternium-37), a methacryloylethyldimethylbetaine-methacryloylethyltrimethylammonium chloride-methoxypolyethylene glycol methacrylate copolymer (polyquaternium-49), a methacryloylethyldimethylbetaine-methacryloylethyltrimethylammonium chloride-2-hydroxyethyl methacrylate copolymer (polyquaternium-48), and an N,N-dimethylaminoethylmethacrylic acid diethyl sulfate-N,N-dimethylacrylamide-polyethylene glycol dimethacrylate copolymer (polyquaternium-52), even more preferably one or more selected from the group consisting of a methacryloylethyltrimethylammonium chloride polymer (polyquaternium-37) and an N,N-dimethylaminoethylmethacrylic acid diethyl sulfate-N,N-dimethylacrylamide-polyethylene glycol dimethacrylate copolymer (polyquaternium-52).
<8> The composition according to any one of <1> to <7>, wherein the component (B) contains (B1) one or more selected from the group consisting of a cross-linked polymer containing a constituent unit derived from (meth)acrylic acid and an anionic polysaccharide.
<9> The composition according to <8>, wherein the crosslinked polymer containing a constituent unit derived from (meth)acrylic acid is a homopolymer or copolymer of (meth)acrylic acid having a crosslinked structure, or a salt thereof.
<10> The composition according to <10>, wherein the homopolymer of (meth)acrylic acid is one or more selected from the group consisting of a polyacrylic acid, a polymethacrylic acid, and a salt thereof.
<11> The composition according to <10>, wherein the copolymer of (meth)acrylic acid is one or more selected from the group consisting of a (meth)acrylic acid/maleic acid copolymer, a (meth)acrylic acid/itaconic acid copolymer, a (meth)acrylic acid/fumaric acid copolymer, a (meth)acrylic acid/vinyl acetate copolymer, a (meth)acrylic acid/(meth)acrylic acid alkyl ester copolymer, a (meth)acrylic acid/2-hydroxyethyl methacrylate copolymer, an acrylic acid/acrylic acid alkyl ester/(N-alkyl)acrylamide copolymer, and a salt thereof, preferably a (meth)acrylic acid/(meth)acrylic acid alkyl ester, more preferably an acrylic acid/acrylic acid alkyl ester.
<12> The composition according to any one of <8> to <11>, wherein the cross-linked polymer containing a constituent unit derived from (meth)acrylic acid is one or more selected from the group consisting of a carboxyvinyl polymer or a salt thereof, an (acrylates/alkyl acrylate (C10-30)) crosspolymer, a (Na acrylate/acryloyldimethyltaurine/dimethylacrylamide)crosspolymer, and an acrylates crosspolymer-4.
<13> The composition according to any one of <8> to <12>, wherein the anionic polysaccharide is one or more selected from the group consisting of a polysaccharide having a carboxy group, a sulfate of a polysaccharide, and a salt thereof, preferably a polysaccharide having a carboxy group or a salt thereof, more preferably alginic acid, carboxymethyl cellulose, or a salt thereof, even more preferably alginic acid or a salt thereof.
<14> The composition according to any one of <8> to <13>, wherein the component (B1) is preferably one or more selected from the group consisting of a carboxyvinyl polymer, an (acrylates/alkyl acrylate (C10-30)) crosspolymer, and an anionic polysaccharide, more preferably an anionic polysaccharide or a salt thereof, and even more preferably alginic acid or a salt thereof.
<15> The composition according to any one of <8> to <14>, wherein the component (B1) has an anionic charge density of preferably 1.0 mmol/g or more and 25 mmol/g or less, more preferably 2.0 mmol/g or more and 20 mmol/g or less, even more preferably 3.5 mmol/g or more and 15 mmol/g or less.
<16> The composition according to any one of <8> to <15>, wherein the anionic polysaccharide as the component (B1) has a weight average molecular weight (Mw) of preferably 2000 or more and 3500000 or less, more preferably 10000 or more and 3000000 or less, even more preferably more than 30000 and 3000000 or less, and even more preferably 50000 or more and 3000000 or less.
<17> The composition according to any one of <8> to <16>, wherein a content of the component (B1) in the component (B) is preferably 50 mass% or more, more preferably 60 mass% or more, even more preferably 70 mass% or more, even more preferably 80 mass% or more, and is 100 mass% or less.
<18> The composition according to any one of <8> to <17>, wherein the component (B) further contains (B2) an anionic polymer other than the component (B1).
<19> The composition according to <18>, wherein the component (B2) has a molecular weight of preferably 1000 or more and 50000 or less, more preferably 1000 or more and 30000 or less, even more preferably 2000 or more and 30000 or less, even more preferably 5000 or more and 30000 or less, and even more preferably 10000 or more and 30000 or less.
<20> The composition according to <18> or <19>, wherein the component (B2) is preferably a polymer having a carboxy group with a molecular weight of 1000 or more and 50000 or less, more preferably 1000 or more and 30000 or less, and more preferably a non-crosslinked polymer containing a constituent unit derived from (meth)acrylic acid with a molecular weight of 1000 or more and 30000 or less.
<21> The composition according to <20>, wherein the non-crosslinked polymer containing a constituent unit derived from (meth)acrylic acid is one or more selected from the group consisting of a poly(meth)acrylic acid, a (meth)acrylic acid/maleic acid copolymer, a (meth)acrylic acid/itaconic acid copolymer, a (meth)acrylic acid/fumaric acid copolymer, a (meth)acrylic acid/vinyl acetate copolymer, a (meth)acrylic acid/(meth)acrylic acid alkyl ester copolymer, a (meth)acrylic acid/2-hydroxyethyl methacrylate copolymer, an acrylic acid/acrylic acid alkyl ester/(N-alkyl) acrylamide copolymer, and a salt thereof.
<22> The composition according to any one of <18> to <21>, wherein the component (B2) is one or more selected from the group consisting of a poly(meth)acrylic acid, a (meth)acrylic acid/(meth)acrylic acid alkyl ester copolymer, and a salt thereof.
<23> The composition according to any one of <18> to <22>, wherein the component (B2) has an anionic charge density of preferably 1.0 mmol/g or more and 25 mmol/g or less, more preferably 3.0 mmol/g or more and 20 mmol/g or less, even more preferably 5.0 mmol/g or more and 15 mmol/g or less.
<24> The composition according to any one of <18> to <23>, wherein a content of the component (B2) in the component (B) is preferably 1 mass% or more and 50 mass% or less, more preferably 2 mass% or more and 40 mass% or less, even more preferably 3 mass% or more and 30 mass% or less, even more preferably 5 mass% or more and 20 mass% or less.
<25> The composition according to any one of <18> to <24>, wherein a mass ratio [(B1)/(B2) + (B2)}] of the component (B2) to a total amount of the component (B1) and the component (B2) is preferably 0.01 or more and 1.0 or less, more preferably 0.02 or more and 0.80 or less, even more preferably 0.05 or more and 0.50 or less, and even more preferably 0.08 or more and 0.30 or less.
<26> The composition according to any one of <1> to <25>, wherein the polyglycerol-modified silicone preferably has a polyglycerol chain in a side chain or at a terminal of a silicone chain, more preferably is a silicone having a monovalent polyglycerol group in a side chain or at a terminal of a silicone chain, and even more preferably is one or more selected from the group consisting of polyglyceryl-3 polydimethylsiloxyethyl dimethicone, polyglyceryl-3 disiloxane dimethicone, lauryl polyglyceryl-3 polydimethylsiloxyethyl dimethicone, cetyl diglyceryl tris(trimethylsiloxy)silylethyl dimethicone, and bis(polyglyceryl-3-oxyphenylpropyl)dimethicone.
<27> The composition according to any one of <1> to <26>, wherein the amino-modified silicone is a silicone represented by General Formula (2) described below: 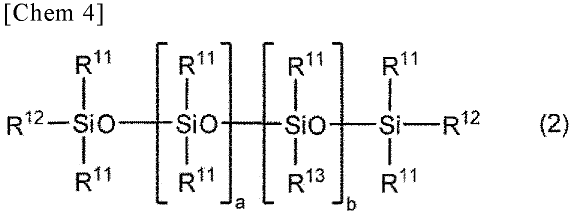 wherein R¹¹ each independently represents a methyl group or a phenyl group, and R¹² each independently represents an alkyl group having 1 or more and 30 or less carbons, a hydroxy group, or R¹³, R¹³ represents a monovalent group represented by -R¹⁴-Z¹ (R¹⁴ represents a single bond or a divalent hydrocarbon group having 1 or more and 20 or less carbons, and Z¹ represents a primary to tertiary amino group-containing group or an ammonium group-containing group), a represents the number of 0 or more and 3000 or less, b represents the number of 1 or more and 3000 or less, and a + b is the number of 100 or more and 20000 or less.
<28> The composition according to <27>, wherein the silicone represented by General Formula (2) is one or more selected from the group consisting of aminoethylaminopropyl dimethicone [amodimethicone], aminopropyl dimethicone, bis(aminopropyl)dimethicone, and bis(cetearyl)amodimethicone.
<29> The composition according to any one of <1> to <28>, wherein the component (C) is an amino-modified silicone.
<30> The composition according to any one of <1> to <29>, wherein the component (D) has a viscosity at 25°C of preferably 500 mm²/s or more, more preferably 700 mm²/s or more, even more preferably 1000 mm²/s or more.
<31> The composition according to any one of <1> to <30>, wherein the component (D) contains (D1) a dimethylpolysiloxane having a viscosity at 25°C of 1000 mm²/s or more and 10000 mm²/s or less and (D2) a dimethylpolysiloxane having a viscosity at 25°C of 300000 mm²/s or more and 5000000 mm²/s or less.
<32> The composition according to <31>, wherein amass ratio [(D2)/(D1)] of the component (D1) to the component (D2) is preferably 0.5 or more and 20 or less, more preferably 1.0 or more and 10 or less, and even more preferably 2.0 or more and 5.0 or less.
<33> The composition according to <31> or <32>, wherein a total content of the component (D1) and the component (D2) in the component (D) is preferably 50 mass% or more, more preferably 60 mass% or more, even more preferably 70 mass% or more, even more preferably 80 mass% or more, even more preferably 90 mass% or more, and is 100 mass% or less.
<34> The composition according to any one of <1> to <33>, wherein the component (E) is preferably one or more selected from the group consisting of (i) an alkyltrimethylammonium salt, (ii) an alkoxyalkyltrimethylammonium salt, (iii) a dialkyldimethylammonium salt, (iv) an alkylamidoalkyltrimethylammonium salt, (v) alkyldimethylamine and a salt thereof, (vi) alkoxyalkyldimethylamine and a salt thereof, and (vii) alkylamidoalkyldimethylamine and a salt thereof, more preferably one or more selected from the group consisting of (i) an alkyltrimethylammonium salt and (vi) alkoxyalkyldimethylamine and a salt thereof, even more preferably one or more selected from the group consisting of cetyltrimethylammonium chloride, stearyltrimethylammonium chloride, behenyltrimethylammonium chloride, N,N-dimethyl-3-hexadecyloxypropylamine or a lactate salt thereof, and N,N-dimethyl-3-octadecyloxypropylamine (stearoxypropyldimethylamine) or a lactate salt thereof, and even more preferably one or more selected from the group consisting of behenyltrimethylammonium chloride and N,N-dimethyl-3-octadecyloxypropylamine (stearoxypropyldimethylamine) or a lactate salt thereof.
<35> The composition according to any one of <1> to <34>, wherein the component (F) is one or more selected from the group consisting of lauryl alcohol, cetyl alcohol, myristyl alcohol, oleyl alcohol, stearyl alcohol, isostearyl alcohol, 2-octyldodecanol, behenyl alcohol and cetostearyl alcohol, preferably one or more selected from the group consisting of cetyl alcohol, myristyl alcohol, stearyl alcohol and behenyl alcohol.
<36> The composition according to any one of <1> to <35>, wherein the composition further contains (G) a nonionic polymer.
<37> The composition according to <36>, wherein the component (G) is one or more selected from the group consisting of polyalkylene glycol or a derivative thereof, a polymer containing a constituent unit derived from vinylpyrrolidone, a polymer containing a constituent unit derived from a (meth)acrylic acid ester, polyvinyl alcohol, polyacrylamide, and polycaprolactam.
<38> The composition according to <37>, wherein the polyalkylene glycol is preferably one or more selected from the group consisting of polyethylene glycol, polypropylene glycol, and a polyethylene glycol-polypropylene glycol copolymer, and more preferably one or more selected from the group consisting of polyethylene glycol and a polyethylene glycol-polypropylene glycol copolymer.
<39> The composition according to <37>, wherein the polyalkylene glycol derivative is one or more selected from the group consisting of a polyalkylene glycol monoalkyl ether, a polyalkylene glycol dialkyl ether, a polyalkylene glycol monoaryl ether, a polyalkylene glycol diaryl ether, a polyalkylene glycol monofatty acid ester, a polyalkylene glycol difatty acid ester, and a polyalkylene glycol diglycidyl ether, preferably one or more selected from the group consisting of a polyalkylene glycol monoalkyl ether, a polyalkylene glycol dialkyl ether, a polyalkylene glycol monofatty acid ester, and a polyalkylene glycol difatty acid ester, more preferably one or more selected from the group consisting of a polyalkylene glycol monofatty acid ester and a polyalkylene glycol difatty acid ester.
<40> The composition according to any one of <36> to <39>, wherein the component (G) is preferably one or more selected from the group consisting of polyalkylene glycol or a derivative thereof and a polymer containing a constituent unit derived from vinylpyrrolidone, more preferably one or more selected from the group consisting of polyethylene glycol, polypropylene glycol, a polyethylene glycol-polypropylene glycol copolymer, a mono- or difatty acid ester thereof, and polyvinylpyrrolidone, and even more preferably one or more selected from the group consisting of polyethylene glycol, a polyethylene glycol-polypropylene glycol copolymer, a mono- or di-fatty acid ester thereof, and polyvinylpyrrolidone.
<41> The composition according to any one of <36> to <40>, wherein the component (G) has a weight average molecular weight of preferably 2000 or more and 2000000 or less, more preferably 5000 or more and 1500000 or less, and even more preferably 10000 or more and 1500000 or less.
<42> The composition according to any one of <1> to <41>, wherein the composition further contains (H) an acid.
<43> The composition according to <42>, wherein the component (H) is preferably an organic acid, more preferably one or more selected from the group consisting of a carboxylic acid compound and a sulfonic acid compound other than the component (B).
<44> The composition according to <43>, wherein the organic acid is preferably one or more selected from the group consisting of an aliphatic dicarboxylic acid, a hydroxycarboxylic acid, and an aromatic sulfonic acid, more preferably one or more selected from the group consisting of succinic acid, lactic acid, malic acid, glycolic acid, p-toluenesulfonic acid, and naphthalenesulfonic acid, even more preferably one or more selected from the group consisting of succinic acid and lactic acid, and even more preferably lactic acid.
<45> The composition according to any one of <1> to <44>, wherein the composition further contains water.
<46> The composition according to any one of <1> to <45>, wherein a content of the component (A) in the composition is preferably 0.01 mass% or more and 3.0 mass% or less, more preferably 0.02 mass% or more and 2.5 mass% or less, even more preferably 0.05 mass% or more and 2.0 mass% or less, even more preferably 0.1 mass% or more and 1.5 mass% or less, even more preferably 0.2 mass% or more and 1.5 mass% or less, and even more preferably 0.2 mass% or more and 1.0 mass% or less.
<47> The composition according to any one of <1> to <46>, wherein a content of the component (B) in the composition is preferably 0.005 mass% or more and 3.0 mass% or less, more preferably 0.01 mass% or more and 2.0 mass% or less, even more preferably 0.02 mass% or more and 1.5 mass% or less, even more preferably 0.02 mass% or more and 1.0 mass% or less, even more preferably 0.02 mass% or more and 0.5 mass% or less, even more preferably 0.02 mass% or more and 0.3 mass% or less, even more preferably 0.02 mass% or more and 0.3 mass% or less, even more preferably 0.02 mass% or more and 0.2 mass% or less, even more preferably 0.02 mass% or more and 0.1 mass% or less, and even more preferably 0.025 mass% or more and 0.1 mass% or less.
<48> The composition according to any one of <1> to <47>, wherein a total content of the component (A) and the component (B) in the composition is preferably 0.02 mass% or more and 5.0 mass% or less, more preferably 0.04 mass% or more and 4.0 mass% or less, even more preferably 0.05 mass% or more and 3.0 mass% or less, even more preferably 0.1 mass% or more and 2.0 mass% or less, even more preferably 0.2 mass% or more and 2.0 mass% or less, and even more preferably 0.3 mass% or more and 2.0 mass% or less.
<49> The composition according to any one of <1> to <48>, wherein a mass ratio [(A)/(B)] of the component (A) to the component (B) in the composition is preferably 4.5 or more and 20 or less, more preferably 4.7 or more and 18 or less, even more preferably 4.8 or more and 18 or less, even more preferably 5.0 or more and 18 or less, even more preferably 5.5 or more and 16 or less, even more preferably 6.0 or more and 16 or less, even more preferably 6.2 or more and 15 or less, and even more preferably 6.5 or more and 15 or less.
<50> The composition according to any one of <1> to <49>, wherein a ratio of the number of moles of cationic charges of the component (A) to the number of moles of anionic charges of the component (B) (cationic charges of component (A)/anionic charges of component (B)) is preferably 10/90 to 95/5, more preferably 20/80 to 95/5, even more preferably 25/75 to 90/10, even more preferably 30/70 to 90/10, even more preferably 40/60 to 90/10, even more preferably 50/50 to 90/10, even more preferably 52/48 to 90/10.
<51> The composition according to any one of <1> to <50>, wherein a content of the component (C) in the composition is preferably 0.01 mass% or more and 5.0 mass% or less, more preferably 0.02 mass% or more and 3.0 mass% or less, even more preferably 0.05 mass% or more and 2.0 mass% or less, and even more preferably 0.1 mass% or more and 2.0 mass% or less.
<52> The composition according to any one of <1> to <51>, wherein a content of the component (D) in the composition is preferably 0.05 mass% or more and 15 mass% or less, more preferably 0.1 mass% or more and 10 mass% or less, even more preferably 0.3 mass% or more and 8.0 mass% or less, and even more preferably 0.5 mass% or more and 8.0 mass% or less.
<53> The composition according to any one of <1> to <52>, wherein a total content of the component (C) and the component (D) in the composition is preferably 0.06 mass% or more and 20 mass% or less, more preferably 0.1 mass% or more and 15 mass% or less, even more preferably 0.3 mass% or more and 10 mass% or less, even more preferably 0.5 mass% or more and 10 mass% or less, and even more preferably 0.6 mass% or more and 10 mass% or less.
<54> The composition according to any one of <1> to <53>, wherein a mass ratio [(C)/(D)] of the component (C) to the component (D) in the composition is preferably 0.02 or more and 20 or less, more preferably 0.02 or more and 10 or less, even more preferably 0.05 or more and 5.0 or less, even more preferably 0.05 or more and 2.0 or less, even more preferably 0.1 or more and 1.0 or less, and even more preferably 0.1 or more and 0.5 or less.
<55> The composition according to any one of <1> to <54>, wherein a content of the component (E) in the composition is preferably 0.1 mass% or more and 10 mass% or less, more preferably 0.2 mass% or more and 5.0 mass% or less, and even more preferably 0.5 mass% or more and 3.5 mass% or less.
<56> The composition according to any one of <1> to <55>, wherein a content of the component (F) in the composition is preferably 0.1 mass% or more and 15 mass% or less, more preferably 0.2 mass% or more and 12 mass% or less, even more preferably 0.5 mass% or more and 12 mass% or less, and even more preferably 1.0 mass% or more and 10 mass% or less.
<57> The composition according to any one of <1> to <56>, wherein a total content of the components (A) to (F) in the composition is preferably 1.0 mass% or more and 50 mass% or less, more preferably 2.0 mass% or more and 40 mass% or less, even more preferably 3.0 mass% or more and 30 mass% or less, even more preferably 5.0 mass% or more and 25 mass% or less, and even more preferably 5.0 mass% or more and 21.5 mass% or less.
<58> The composition according to any one of <36> to <57>, wherein a content of the component (G) in the composition is preferably 0.01 mass% or more and 3.0 mass% or less, more preferably 0.02 mass% or more and 2.5 mass% or less, even more preferably 0.05 mass% or more and 2.0 mass% or less, even more preferably 0.1 mass% or more and 1.5 mass% or less, and even more preferably 0.1 mass% or more and 1.0 mass% or less.
<59> The composition according to any one of <36> to <58>, wherein a mass ratio [(G)/{(A) + (B)}] of the component (G) to a total amount of the component (A) and the component (B) in the composition is preferably 0.2 or more and 20 or less, more preferably 0.3 or more and 15 or less, even more preferably 0.4 or more and 10 or less, even more preferably 0.5 or more and 8.0 or less, even more preferably 0.6 or more and 5.0 or less, even more preferably 0.8 or more and 2.0 or less, and even more preferably 0.8 or more and 1.5 or less.
<60> The composition according to any one of <42> to <59>, wherein a content of the component (H) in the composition is preferably 0.01 mass% or more and 5.0 mass% or less, more preferably 0.02 mass% or more and 3.0 mass% or less, even more preferably 0.03 mass% or more and 3.0 mass% or less, even more preferably 0.05 mass% or more and 3.0 mass% or less, even more preferably 0.05 mass% or more and 3.0 mass% or less, even more preferably 0.1 mass% or more and 3.0 mass% or less, even more preferably 0.1 mass% or more and 2.5 mass% or less, and even more preferably 0.2 mass% or more and 2.5 mass% or less.
<61> The composition according to any one of <45> to <60>, wherein the content of the water in the composition is preferably 10 mass% or more and 99 mass% or less, more preferably 20 mass% or more and 95 mass% or less, and even more preferably 30 mass% or more and 90 mass% or less.
<62> The composition according to any one of <1> to <61>, wherein an aqueous solution obtained by diluting the composition by 20 times with water has a pH at 25°C of preferably 3.0 or more, more preferably 3.2 or more, and preferably 7.5 or less, more preferably 7.0 or less, even more preferably 6.0 or less, and even more preferably 5.0 or less.
<63> The composition according to any one of <1> to <62>, wherein the cosmetic composition is preferably a cosmetic composition for keratin materials such as skin, hair, eyelashes, eyebrows, and nails, and more preferably a cosmetic composition for skin or hair.
<64> The composition according to <63>, wherein the skin cosmetic composition has a product form of a body soap, a facial cleanser, a bath agent, a deodorant preparation, a makeup cosmetic, a sunscreen, a makeup foundation, a milky lotion, a beauty serum, or a cream.
<65> The composition according to <63>, wherein the hair cosmetic composition has a product form of a hair shampoo, a hair rinse, a hair conditioner, a hair treatment, a hair styling agent, a hair color, or a permanent agent, and preferably a hair conditioner, a hair treatment, or a hair styling agent.
<66> The composition according to any one of <1> to <65>, wherein the composition has a form of preferably an emulsion composition, more preferably an oil-in-water emulsion composition.
<67> A method for producing a cosmetic composition or a fiber treatment composition for head decoration products, comprising steps 1 to 3, any one of steps 4-1 and 4-2, and step 5 in this order:
   Step 1: Step of preparing a solution 1 containing (B) an anionic polymer
   Step 2: Step of mixing the solution 1 with (H) an acid or an aqueous solution thereof to prepare a mixture 2 containing (B) an anionic polymer, (H) an acid, and water
   Step 3: Step of mixing the mixture 2 with (A) a cationic polymer to prepare a mixture 3 containing (A) a cationic polymer, (B) an anionic polymer, (H) an acid, and water
   Step 4-1: Step of mixing (I) an aqueous phase containing water with (I) an oily phase containing (D) a cationic surfactant and (E) a higher alcohol to prepare (Ia) an emulsion, and then mixing (Ia) an emulsion with the mixture 3 to prepare an emulsion 4-1
   Step 4-2: Step of mixing the mixture 3 with (I) an aqueous phase containing water to prepare (Ia) an aqueous phase, and then mixing (Ia) an aqueous phase with (I) an oily phase containing (D) a cationic surfactant and (E) a higher alcohol to prepare an emulsion 4-2
   Step 5: Step of mixing the emulsion 4-1 or the emulsion 4-2 with (II) an oily phase containing (C) one or more modified silicones selected from the group consisting of a polyglycerol-modified silicone and an amino-modified silicone and (D) a dimethylpolysiloxane

### Examples

The present invention will be described below through examples, but the present invention is not limited to the scope of the examples. Each measurement and evaluation in Examples were performed by the following methods.

### (Measurement of Viscosity of 1 mass% Aqueous Solution of Cationic Polymer)

A 1 mass% aqueous solution of the cationic polymer (component (A) or component (A')) used in each example was prepared, and the aqueous solution viscosity at 30°C was measured using a B-type viscometer (TV-20 manufactured by Toki Sangyo Co., Ltd.). In the present example, the cationic polymer other than the component (A) was denoted as "component (A')".

### (pH Measurement)

The hair conditioner of each example was diluted by 20 times with water, and the pH at 25°C was measured using a pH meter (F-51, manufactured by Horiba, Ltd.).

### (Measurement of Combing Force After Hair Conditioner Treatment + Two Times of Hair Washing)

(1) "Kao Foam Hair Bleach L" (first agent) 7 mL and "Kao Foam Hair Color d" (second agent) 15 mL manufactured by Kao Corporation were mixed, and the mixture was applied to a tress of Japanese hair having a length of 20 cm and a mass of 30 g. After being left to stand for 20 minutes, it was sufficiently rinsed with warm water at 35 to 40°C (bleaching treatment). The bleaching treatment was repeated four times to prepare a damaged tress for evaluation. The tress was washed with a plain shampoo having the following composition and moistened with water at 15 g ± 0.5 g.
(2) The hair conditioner of each example was applied to the water-containing tress by 1.5 mL and rubbed in by hand, then the tress was allowed to stand for 30 seconds, followed by rinsing with warm water at 35 to 40°C for 15 seconds. Next, the front and back surfaces of the tress were rubbed and washed with the plain shampoo 1 mL for 30 seconds 20 times for each surface and rinsed for 15 seconds.
(3) A plain conditioner 1 mL having the following composition was applied, rubbed in by hand for 30 seconds, and rinsed for 15 seconds. Again, the front and back surfaces of the tress were rubbed and washed with the plain shampoo 1 mL for 30 seconds 20 times for each surface and rinsed for 15 seconds.
(4) The tress was combed with a brush to be completely disentangled, and then the water content of the rinsed tress was adjusted to 15 g ± 0.5 g. The tress was set in a combing force measuring device ("KOT-0303" manufactured by Utsunomiya Seiki.), two brushes (Kao Lunette) were passed through the top of the tress, combing was performed at a constant speed (60 rpm), and the load (g) applied when the combs were passed through to the hair tip was measured. Combing was performed 50 times, and the average value of the combing loads at 50 points in total was taken as the value of "Combing force immediately after treatment". A smaller value of the combing force indicates a better result. The combing load of the damaged tress (containing 15 g ± 0.5 g of water) that was not subjected to the treatment (2) was 2055 g, and when the combing force was 1900 g or less, the hair washing resistance was determined to be good.

### [Plain Shampoo Composition] *The blending amount is an active ingredient amount.

| | (mass%) |
|---|---|
| Sodium polyoxyethylene (2.0) lauryl ether sulfate (*1) : | 15.5 |
| Diethanolamide laurate (*2) : | 2.2 |
| Disodium edetate : | 0.15 |
| Sodium benzoate : | 0.18 |
| Oxybenzone : | 0.03 |
| Phosphoric acid : | 0.07 |
| Sodium chloride : | 0.8 |
| Fragrance : | 0.4 |
| Purified water : | balance |
| Total: | 100.00 |

| | |
|---|---|
| (*1) 42.0 mass% as "EMAL E-27C" (active ingredient amount 27 mass%) manufactured by Kao Corporation (*2) AMINON C-11S (manufactured by Kao Corporation), active ingredient amount 100 mass% | |

### [Composition of Plane Conditioner] *Blending amount is an active ingredient amount.

| | (mass%) |
|---|---|
| Stearoxypropyldimethylamine (*3) | 0.81 |
| Stearyl alcohol (*4) | 3.09 |
| Lactic Acid (*5) | 0.21 |
| Purified water | balance |
| Total | :100.00 |

| | |
|---|---|
| (*3) "FARMIN DM-E80" manufactured by Kao Corporation, active ingredient amount 90 mass% (*4) "KALCOL 8098" manufactured by Kao Corporation, active ingredient amount 100 mass% (*5) "PURAC ULTRAPURE90" manufactured by PURAC Thailand Ltd., active ingredient amount 90 mass% | |

### (Measurement of Combing Force After Hair Conditioner Treatment + Four Times of Hair Washing)

In the above "Measurement of Combing Force After Hair Conditioner Treatment + Two Times of Hair Washing", (1) to (2) were performed, thereafter (3) was performed three times, and then the operation of (4) was performed to measure the combing force.

### (Emulsified State of Hair Conditioner After 2 Days from Blending)

After 2 days from the blending, 1 g of the hair conditioner of each example was dropped on a plastic black underlay, and the appearance when being traced 10 times with a finger and the texture when being crushed with a finger were evaluated according to the following criteria.
A: Aggregated particles were not visually recognized, and the texture was slippery when crushed with a finger
B: Aggregated particles were not visually recognized, but the texture was rough when crushed with a finger
C: Aggregated particles were visually recognized, and the texture was rough when crushed with a finger
D: Separated

### (Moisture Resistance of Hair after Treatment)

"Kao Foam Hair Bleach L" (first agent) 7 mL and "Kao Foam Hair Color d" (second agent) 15 mL manufactured by Kao Corporation were mixed, and the mixture was applied to a tress of Japanese hair having a length of 30 cm and a mass of 20 g. After being left to stand for 20 minutes, it was sufficiently rinsed with warm water at 35 to 40°C (bleaching treatment). The bleaching treatment was repeated four times to prepare a damaged tress for evaluation.

After the tresses were washed with the plain shampoo, the hair conditioner 1 mL of each example was applied and rubbed in for 30 seconds, left to stand in this state for 1 minute, rinsed with running water at 35 to 40°C for 30 seconds, and dried with a dryer until completely dried.

Using a flat iron ("AHI-938" manufactured by Miki Denki Sangyo) set at 160°C, the tress after drying was subjected to straight setting 10 times while a comb was passed through, and a photograph of the tress immediately after setting was taken. Next, the hair after straight setting was suspended and left to stand for 30 minutes under a high-humidity environment of 35°C and 80% RH, and a photograph was taken again. In the two photographs, the tress width at the 23.5 cm from the root of the tress was measured, and the change in the tress width between immediately after straight setting and after high-humidity storage was calculated from the following equation. Tress width change value = (tress width after storage under high humidity)/(tress width immediately after straight setting)

When the value was 1.3 or more, it appeared to be spread, and thus it was determined that the moisture resistance was low.

### Examples 1 to 13 and Comparative Examples 1 to 11 (Preparation and Evaluation of Hair Conditioner)

A hair conditioner was prepared by the following procedure using the components in the amounts listed in the table.

An anionic polymer (component (B1), or component (B1) and component (B2)) dispersed in dipropylene glycol in advance was added to a 500 mL beaker containing water, and the mixture was heated and stirred at 56°C. It was confirmed that the anionic polymer was completely dissolved, and in Examples 2 to 9 and 12 to 13, (G) a nonionic polymer was further added and completely dissolved.

Then 90% lactic acid (57% of the total amount) and cationic polymer (component (A) or component (A')) were added. Further, (E) a cationic surfactant, (F) a higher alcohol, the remaining dipropylene glycol and benzyl alcohol were mixed in advance and dissolved at 85°C to obtain an oily phase, which was then added and stirred at 56°C for 10 minutes. After stirring for 10 minutes, heating was terminated, and air cooling was started. After air cooling, the silicone (components (C) and (D)) shown in the table and the remaining 90% lactic acid were added, and the mixture was cooled with stirring until the liquid temperature reached 40°C or less to obtain a hair conditioner in an amount of 500 mL.

The obtained hair conditioner was subjected to various evaluations according to the methods described above. The results are shown in Tables 1 to 4.

### [Table 1]

**Table 1**

| (mass%) SOFCARE KG-101W-E (charge | | | Examples | | | | | | |
|---|---|---|---|---|---|---|---|---|---|
| | | | 1 | 2 | 3 | 4 | 5 | 6 | 7 |
| (A) | Polyquaternium-52 | density 0.83 mmol/g) *1 (1% viscosity 2560 mPa·s) active 2.4% | 0.353 | 0.353 | 0.353 | 0.353 | 0.355 | | |
| | Polyquaternium-52 | SOFCARE KG-301W (charge density 1.84 mmol/g) *2 (1% viscosity 2070 mPa·s) active 4.02% | | | | | | 0.353 | |
| | Polyquaternium-37 | Ultragel 300 (charge density 4.81 mmol/g) *3 (1% viscosity 17650 mPa s) | | | | | | | 0.353 |
| (A') | Polyquaternium-22 | Merquat 295 (charge density 6.04 mmol/g) *4 (1% viscosity 5.6 mPa s) | | | | | | | |
| | Polyquaternium-55 | STYLEZE W17 (charge density unknown) *5 (1% viscosity 6.0 mPa s) | | | | | | | |
| (B1) | Sodium alginate | KIMICA ALGIN I-3-150 *6 (charge density 4.65 mmol/g, 1% viscosity 300 to 400 mPa·s) | 0.041 | 0.041 | 0.041 | 0.041 | 0.042 | 0.041 | 0.041 |
| | Sodium alginate | KIMICA ALGIN IL-2 *7 (charge density 4.65 mmol/g, 1% viscosity 20 to 50 mPa s) | | | | | | | |
| (B2) | (Acrylic acid/stearyl acrylate) copolymer | SOFCARE SA-37W *8 (charge density 8.88 mmol/g, Mw 20000) | | | | 0.006 | | 0.006 | 0.006 |
| | Polyacrylic acid | Jurymer AC-10L *9 (charge density 13.88 mmol/g, Mw 20000 to 30000) | | | | | 0.004 | | |
| | Polyacrylic acid | Polyacrylic acid (Mw 25000) *10 | | | | | | | |
| | Polyacrylic acid | Polyacrylic acid (Mw 5000) *11 | | | | | | | |
| (C) | Amino-modified silicone having a viscosity of 100 mm2/s or more and 5000 mm2/s or less | SF8457C *12 | 0.176 | 0.176 | 0.176 | 0.176 | 0.176 | 0.176 | 0.176 |
| | Polyglycerol-modified silicone having a viscosity of 100 mm2/s or more and 5000 mm2/s or less | SOFCARE GS-G *13 | | | | | | | |
| (C') | Aminopolyether-modified silicone having a viscosity of 100 mm2/s or more and 5000 mm2/s or less | Silicone SILSTYLE104 *14 | | | | | | | |
| (D) | (D1) Dimethylpolysiloxane having a viscosity of 1000 mm2/s or more and 10000 mm2/s or less | 5000cs silicone | 0.602 | 0.602 | 0.602 | 0.602 | 0.602 | 0.602 | 0.602 |
| | (D2) Dimethylpolysiloxane having a viscosity of 300000 mm2/s or more and 5000000 mm2/s or less | Highly polymerized silicone | 0.222 | 0.222 | 0.222 | 0.222 | 0.222 | 0.222 | 0.222 |
| | Dimethylpolysiloxane | KHS-3 *15 | | | | | | | |
| (G) | Polyethylene glycol | BLAUNON PEG-20000S *16 | | 0.4 | | 0.4 | 0.4 | 0.4 | 0.4 |
| | Polyvinylpyrrolidone | Luviskol K90 *17 | | | 0.4 | | | | |
| (E) | Stearoxypropyldimethylamine | FARMIN DM-E80 *18 | | | | | | | |
| | sehenyltrimethylammonium chloride | QUARTAMIN 2285-E *19 | 1.2 | 1.2 | 1.2 | 1.2 | 1.2 | 1.2 | 1.2 |
| (F) | Cetanol | KALCOL 6870 *20 | 2.4 | 2.4 | 2.4 | 2.4 | 2.4 | 2.4 | 2.4 |
| (H) | Lactic acid | 90% Lactic acid *21 | Appropriate amount | Appropriate amount | Appropriate amount | Appropriate amount | Appropriate amount | Appropriate amount | Appropriate amount |
| Other Components | Dipropylene glycol | DPG-RF *22 | 4 | 4 | 4 | 4 | 4 | 4 | 4 |
| | Benzyl alcohol | | 0.3 | 0.3 | 0.3 | 0.3 | 0.3 | 0.3 | 0.3 |
| | Water | Ion exchange water | Balance | Balance | Balance | Balance | Balance | Balance | Balance |
| Total | | | 100 | 100 | 100 | 100 | 100 | 100 | 100 |

**Table 1 (continued)**

| (mass%) | | | Examples | | | | | | |
|---|---|---|---|---|---|---|---|---|---|
| | | | 1 | 2 | 3 | 4 | 5 | 6 | 7 |
| pH (25°C, at 20-fold dilution) | | | 3.4 | 3.4 | 3.4 | 3.4 | 3.4 | 3.4 | 3.4 |
| Content of cationic polymer (component (A) or (A')) in composition (mass%) | | | 0.353 | 0.353 | 0.353 | 0.353 | 0.355 | 0.353 | 0.353 |
| Content of anionic polymer (component (B)) in composition (mass%) | | | 0.041 | 0.041 | 0.041 | 0.047 | 0.046 | 0.047 | 0.047 |
| Total content of cationic polymer and anionic polymer in composition (mass%) | | | 0.394 | 0.394 | 0.394 | 0.400 | 0.401 | 0.400 | 0.400 |
| Cation (A) or (A')/anion (B1)/anion (B2) charge ratio (molar ratio) | | | 60/40/0 | 60/40/0 | 60/40/0 | 55/36/9 | 54/36/10 | 72.9/21.5/5.6 | 87.6/9.9/2.6 |
| Mass ratio (A)/(B) (or (A')/(B)) | | | 8.57 | 8.57 | 8.57 | 7.55 | 7.72 | 7.54 | 7.54 |
| Content of component (B1) in component (B) (mass%) | | | 100.00 | 100.00 | 100.00 | 88.03 | 91.30 | 88.03 | 88.03 |
| Content of component (B2) in component (B) (mass%) | | | 0.00 | 000 | 000 | 11.97 | 8.70 | 11.97 | 11.97 |
| Mass ratio (B2)/{(B1) + (B2)} | | | 0.00 | 0.00 | 000 | 0.12 | 0.09 | 0.12 | 0.12 |
| Content of component (C) in composition (mass%) | | | 0.176 | 0.176 | 0.176 | 0.176 | 0.176 | 0.176 | 0.176 |
| Content of component (D) in composition (mass%) | | | 0.824 | 0.824 | 0.824 | 0.824 | 0.824 | 0.824 | 0.824 |
| Total content of component (C) and component (D) in composition (mass%) | | | 1.000 | 1.000 | 1.000 | 1.000 | 1.000 | 1.000 | 1.000 |
| Mass ratio (C)/(D) | | | 0.21 | 0.21 | 0.21 | 0.21 | 0.21 | 0.21 | 0.21 |
| Total content of components (A) to (F) in composition (mass%) | | | 4.99 | 4.99 | 4.99 | 5.00 | 5.00 | 5.00 | 5.00 |
| Content of component (G) in composition (mass%) | | | 0.00 | 0.40 | 0.40 | 0.40 | 0.40 | 0.40 | 0.40 |
| Mass ratio (G)/{(A) + (B)} (or (G)/{(A') + (B)}) | | | 0.00 | 1.01 | 1.01 | 1.00 | 1.00 | 1.00 | 1.00 |
| Effect | Combing force after hair conditioner treatment + two times of hair washing | Standard: 1900 g or less *blank = 2055 g | 1366 | 1395 | 1621 | 1333 | 1550 | 1286 | 1351 |
| | Combing force after hair conditioner treatment + four times of hair washing | | 1858 | 1572 | | 1395 | | | |
| | Emulsified state of hair conditioner after 2 days from blending | A: Aggregated particles cannot be visually recognized, and slippery when crushed | A | A | A | A | A | A | A |
| | | B: Aggregated particles cannot be visually recognized, but rough when crushed. | | | | | | | |
| | | C: Aggregated particles can be visually recognized, and are rough when crushed. | | | | | | | |
| | | D: Cd is separated | | | | | | | |
| | Moisture resistance of hair after treatment | Standard: less than 1.3 | 1.18 | 1.16 | 1.06 | 1.19 | 1.17 | 1.14 | 1.13 |

### [Table 2]

**Table 2**

| (mass%) | | | Examples | | | | | |
|---|---|---|---|---|---|---|---|---|
| | | | 8 | 9 | 10 | 11 | 12 | 13 |
| (A) | Polyquaternium-52 | SOFCARE KG-101W-E (charge density 0.83 mmol/g) *1 (1% viscosity 2560 mPa s) active 2.4% | 0.353 | 0.353 | 0.353 | 0.353 | 0.343 | 0.370 |
| | Polyquaternium-52 | SOFCARE KG-301W (charge density 1.84 mmol/g) *2 (1% viscosity 2070 mPa s) active 4.02% | | | | | | |
| | Polyquaternium-37 | Ultragel 300 (charge density 4.81 mmol/g) *3 (1% viscosity 17650 mPa s) | | | | | | |
| (A') | Polyquaternium-22 | Merquat 295 (charge density 6.04 mmol/g) *4 (1% viscosity 5.6 mPa s) | | | | | | |
| | Polyquaternium-55 | STYLEZE W17 (charge density unknown) *5 (1% viscosity 6.0 mPa s) | | | | | | |
| (B1) | Sodium alginate | KIMICA ALGIN I-3-150 *6 (charge density 4.65 mmol/g, 1% viscosity 300 to 400 mPa s) | | 0.041 | 0.041 | 0.041 | 0.052 | 0.025 |
| | Sodium alginate | KIMICA ALGIN IL-2 *7 (charge density 4.65 mmol/g, 1% viscosity 20 to 50 mPa s) | 0.041 | | | | | |
| (B2) | (Acrylic acid/stearyl acrylate) copolymer | SOFCARE SA-37W *8 (charge density 8.88 mmol/g. Mw 20000) | 0.006 | 0.006 | | | | |
| | Polyacrylic acid | Jurymer AC-10L *9 (charge density 13.88 mmol/g, Mw 20000 to 30000) | | | | | | |
| | Polyacrylic acid | Polyacrylic acid (Mw 25000) *10 | | | | | | |
| | Polyacrylic acid | Polyacrylic acid (Mw 5000) *11 | | | | | | |
| (C) | Amino-modified silicone having a viscosity of 100 mm2/s or more and 5000 mm2/s or less | SF8457C *12 | 0.176 | 0.176 | 0.75 | | 0.176 | 0.176 |
| | Polyglycerol-modified silicone having a viscosity of 100 mm2/s or more and 5000 mm2/s or less | SOFCARE GS-G *13 | | | | 0.176 | | |
| (C') | Aminopolyether-modified silicone having a viscosity of 100 mm2/s or more and 5000 mm2/s or less | Silicone SILSTYLE104 *14 | | | | | | |
| (D) | (D1) Dimethylpolysiloxane having a viscosity of 1000 mm2/s or more and 10000 mm2/s or less | 5000cs silicone | 0.602 | 0.602 | 0.178 | 0.602 | 0.602 | 0.602 |
| | (D2) Dimethylpolysiloxane having a viscosity of 300000 mm2/s or more and 5000000 mm2/s or less | Highly polymerized silicone | 0.222 | 0.222 | 0.072 | 0.222 | 0.222 | 0.222 |
| | Dimethylpolysiloxane | KHS-3 *15 | | | | | | |
| (G) | Polyethylene glycol | BLAUNON PEG-20000S *16 | 0.4 | 0.4 | | | 0.4 | 0.4 |
| | Polyvinylpyrrolidone | Luviskol K90 *17 | | | | | | |
| (E) | Stearoxypropyldimethylamine | FARMIN DM-E80 *18 | | 1.4 | | | | |
| | Behenyltrimethylammonium chloride | QUARTAMIN 2285-E *19 | 1.2 | | 1.2 | 1.2 | 1.2 | 1.2 |
| (F) | Cetanol | KALCOL 6870 *20 | 2.4 | 3.7 | 2.4 | 2.4 | 2.4 | 2.4 |
| (H) | Lactic acid | 90% Lactic acid *21 | Appropriate amount | Appropriate amount | Appropriate amount | Appropriate amount | Appropriate amount | Appropriate amount |
| Other Components | Dipropylene glycol | DPG-RF *22 | 4 | 4 | 4 | 4 | 4 | 4 |
| | Benzyl alcohol | | 0.3 | 0.3 | 0.3 | 0.3 | 0.3 | 0.3 |
| | Water | Ion exchange water | Balance | Balance | Balance | Balance | Balance | Balance |
| Total | | | 100 | 100 | 100 | 100 | 100 | 100 |

**Table 2 (continued)**

| (mass%) | | | Examples | | | | | |
|---|---|---|---|---|---|---|---|---|
| | | | 8 | 9 | 10 | 11 | 12 | 13 |
| pH (25°C, at 20-fold dilution) | | | 3.4 | 3.4 | 3.4 | 3.4 | 3.4 | 3.4 |
| Content of cationic polymer (component (A) or (A')) in composition (mass%) | | | 0.353 | 0.353 | 0.353 | 0.353 | 0.343 | 0.370 |
| Content of anionic polymer (component (B)) in composition (mass%) | | | 0.047 | 0.047 | 0.041 | 0.041 | 0.052 | 0.025 |
| Total content of cationic polymer and anionic polymer in composition (mass%) | | | 0.400 | 0.400 | 0.394 | 0.394 | 0.395 | 0.395 |
| Cation (A) or (A')/anion (B1)/anion (B2) charge ratio (molar ratio) | | | 55/35/10 | 54/46/0 | 60/40/0 | 60/40/0 | 54/46/0 | 73/27/0 |
| Mass ratio (A)/(B) (or (A')/(B)) | | | 7.51 | 7.48 | 8.57 | 8.57 | 6.63 | 14.88 |
| Content of component (B1) in component (B) (mass%) | | | 87.23 | 87.29 | 100.00 | 100.00 | 100.00 | 100.00 |
| Content of component (B2) in component (B) (mass%) | | | 12.77 | 12.71 | 000 | 0.00 | 0.00 | 000 |
| Mass ratio (B2)/{(B1) + (B2)} | | | 0.13 | 0.13 | 000 | 0.00 | 0.00 | 000 |
| Content of component (C) in composition (mass%) | | | 0.176 | 0.18 | 0.750 | 0.176 | 0.176 | 0.176 |
| Content of component (D) in composition (mass%) | | | 0.824 | 0.82 | 0.250 | 0.824 | 0.824 | 0.824 |
| Total content of component (C) and component (D) in composition (mass%) | | | 1.000 | 1.00 | 1.000 | 1.000 | 1.000 | 1.000 |
| Mass ratio (C)/(D) | | | 0.21 | 021 | 3.00 | 0.21 | 0.21 | 0.21 |
| Total content of components (A) to (F) in composition (mass%) | | | 5.00 | 6.50 | 4.99 | 4.99 | 4.99 | 5.00 |
| Content of component (G) in composition (mass%) | | | 0.40 | 0.40 | 0.00 | 0.00 | 0.40 | 0.40 |
| Mass ratio (G)/{(A) + (B)} (or (G)/{(A') + (B)}) | | | 1.00 | 1.00 | 0.00 | 0.00 | 1.01 | 1.01 |
| Effect | Combing force after hair conditioner treatment + two times of hair washing | Standard: 1900 g or less *blank = 2055 g | 1366 | 1231 | 1409 | 1533 | 1381 | 1393 |
| | Combing force after hair conditioner treatment + four times of hair washing | | | | | | 1868 | 1895 |
| | Emulsified state of hair conditioner after 2 days from blending | A: Aggregated particles cannot be visually recognized, and slippery when crushed | A | A | A | A | A | A |
| | | B: Aggregated particles cannot be visually recognized, but rough when crushed. | | | | | | |
| | | C: Aggregated particles can be visually recognized, and are rough when crushed. | | | | | | |
| | | D: Cd is separated | | | | | | |
| | Moisture resistance of hair after treatment | Standard: less than 1.3 | 1.15 | 1.16 | 1.03 | 1.14 | 1.04 | 1.08 |

### [Table 3]

**Table 3**

| (mass%) | | | Comparative Examples | | | | | |
|---|---|---|---|---|---|---|---|---|
| | | | 1 | 2 | 3 | 4 | 5 | 6 |
| (A) | Polyquaternium-52 | SOFCARE KG-101W-E (charge density 0.83 mmol/g) *1 (1% viscosity 2560 mPa s) active 2.4% | | 0.353 | 0.353 | | 0.353 | 0.353 |
| | Polyquaternium-52 | SOFCARE KG-301W (charge density 1.84 mmol/g) *2 (1% viscosity 2070 mPa s) active 4.02% | | | | | | |
| | Polyquaternium-37 | Ultragel 300 (charge density 4.81 mmol/g) *3 (1% viscosity 17650 mPa s) | | | | | | |
| (A') | Polyquaternium-22 | Merquat 295 (charge density 6.04 mmol/g) *4 (1% viscosity 5.6 mPa s) | | | | | | |
| | Polyquaternium-55 | STYLEZE W17 (charge density unknown) *5 (1% viscosity 6.0 mPa s) | | | | | | |
| (B1) | Sodium alginate | KIMICA ALGIN I-3-150 *6 (charge density 4.65 mmol/g, 1% viscosity 300 to 400 mPa s) | 0.041 | | 0.041 | | 0.041 | 0.041 |
| | Sodium alginate | KIMICA ALGIN IL-2 *7 (charge density 4.65 mmol/g, 1% viscosity 20 to 50 mPa s) | | | | | | |
| (B2) | (Acrylic acid/stearyl acrylate) copolymer | SOFCARE SA-37W *8 (charge density 8.88 mmol/g. Mw 20000) | | | | | | |
| | Polyacrylic acid | Jurymer AC-10L *9 (charge density 13.88 mmol/g, Mw 20000 to 30000) | | | | | | |
| | Polyacrylic acid | Polyacrylic acid (Mw 25000) *10 | | | | | | |
| | Polyacrylic acid | Polyacrylic acid (Mw 5000) *11 | | | | | | |
| (C) | Amino-modified silicone having a viscosity of 100 mm2/s or more and 5000 mm2/s or less | SF8457C *12 | 0.176 | 0.176 | | 0.176 | | 1.0 |
| | Polyglycerol-modified silicone having a viscosity of 100 mm2/s or more and 5000 mm2/s or less | SOFCARE GS-G *13 | | | | | | |
| (C') | Aminopolyether-modified silicone having a viscosity of 100 mm2/s or more and 5000 mm2/s or less | Silicone SILSTYLE104 *14 | | | | | | |
| (D) | (D1) Dimethylpolysiloxane having a viscosity of 1000 mm2/s or more and 10000 mm2/s or less | 5000cs silicone | 0.602 | 0.602 | | 0.602 | | |
| | (D2) Dimethylpolysiloxane having a viscosity of 300000 mm2/s or more and 5000000 mm2/s or less | Highly polymerized silicone | 0.222 | 0.222 | | 0.222 | | |
| | Dimethylpolysiloxane | KHS-3 *15 | | | | | 1.0 | |
| (G) | Polyethylene glycol | BLAUNON PEG-20000S *16 | | | | | | |
| | Polyvinylpyrrolidone | Luviskol K90 *17 | | | | | | |
| (E) | Stearoxypropyldimethylamine | FARMIN DM-E80 *18 | | | | | | |
| | Behenyltrimethylammonium chloride | QUARTAMIN 2285-E *19 | 1.2 | 1.2 | 1.2 | 1.2 | 1.2 | 1.2 |
| (F) | Cetanol | KALCOL 6870 *20 | 2.4 | 2.4 | 2.4 | 2.4 | 2.4 | 2.4 |
| (H) | Lactic acid | 90% Lactic acid *21 | Appropriate amount | Appropriate amount | Appropriate amount | Appropriate amount | Appropriate amount | Appropriate amount |
| Other Components | Dipropylene glycol | DPG-RF *22 | 4 | 4 | 4 | 4 | 4 | 4 |
| | Benzyl alcohol | | 0.3 | 0.3 | 0.3 | 0.3 | 0.3 | 0.3 |
| | Water | Ion exchange water | Balance | Balance | Balance | Balance | Balance | Balance |
| Total | | | 100 | 100 | 100 | 100 | 100 | 100 |

**Table 3 (continued)**

| (mass%) | | | Comparative Examples | | | | | |
|---|---|---|---|---|---|---|---|---|
| | | | 1 | 2 | 3 | 4 | 5 | 6 |
| pH (25°C, at 20-fold dilution) | | | 3.4 | 3.4 | 3.4 | 3.4 | 3.4 | 3.4 |
| Content of cationic polymer (component (A) or (A')) in composition (mass%) | | | 0.000 | 0.353 | 0.353 | 0.000 | 0.353 | 0.353 |
| Content of anionic polymer (component (B)) in composition (mass%) | | | 0.041 | 0.000 | 0.041 | 0.000 | 0.041 | 0.041 |
| Total content of cationic polymer and anionic polymer in composition (mass%) | | | 0.041 | 0.353 | 0.394 | 0.000 | 0.394 | 0.394 |
| Cation (A) or (A')/anion (B1)/anion (B2) charge ratio (molar ratio) | | | 0/100/0 | 100/0/0 | 61/39/0 | - | 60/40/0 | 55/36/9 |
| Mass ratio (A)/(B) (or (A')/(B)) | | | 0.00 | - | 8.61 | - | 8.57 | 8.57 |
| Content of component (B1) in component (B) (mass%) | | | 100.00 | - | 100.00 | - | 100.00 | 100.00 |
| Content of component (B2) in component (B) (mass%) | | | 0.00 | - | 000 | - | 0.00 | 000 |
| Mass ratio (B2)/{(B1) + (B2)} | | | 0.00 | - | 0.00 | - | 0.00 | 000 |
| Content of component (C) in composition (mass%) | | | 0.176 | 0.176 | 0.000 | 0.176 | 0.000 | 1.000 |
| Content of component (D) in composition (mass%) | | | 0.824 | 0.824 | 0.000 | 0.824 | 1.000 | 0.000 |
| Total content of component (C) and component (D) in composition (mass%) | | | 1.000 | 1.000 | 0.000 | 1.000 | 1.000 | 1.000 |
| Mass ratio (C)/(D) | | | 0.21 | 021 | - | 0.21 | 000 | - |
| Total content of components (A) to (F) in composition (mass%) | | | 4.64 | 4.95 | 3.99 | 4.60 | 4.99 | 4.99 |
| Content of component (G) in composition (mass%) | | | 0.00 | 000 | 0.00 | 0.00 | 0.00 | 000 |
| Mass ratio (G)/{(A) + (B)} (or (G)/{(A') + (B)}) | | | 0.00 | 000 | 0.00 | - | 0.00 | 000 |
| Effect | Combing force after hair conditioner treatment + two times of hair washing | Standard: 1900 g or less *blank = 2055 g | 1972 | 2025 | 1994 | 2044 | 1970 | 2024 |
| | Combing force after hair conditioner treatment + four times of hair washing | | | | | | | |
| | Emulsified state of hair conditioner after 2 days from blending | A: Aggregated particles cannot be visually recognized, and slippery when crushed | A | A | A | A | A | A |
| | | B: Aggregated particles cannot be visually recognized, but rough when crushed. | | | | | | |
| | | C: Aggregated particles can be visually recognized, and are rough when crushed. | | | | | | |
| | | D: Cd is separated | | | | | | |
| | Moisture resistance of hair after treatment | Standard: less than 1.3 | 1.39 | 1.30 | 1.24 | 1.30 | 1.22 | 1.24 |

### [Table 4]

**Table 4**

| (mass%) | | | Comparative Examples | | | | |
|---|---|---|---|---|---|---|---|
| | | | 7 | 8 | 9 | 10 | 11 |
| (A) | Polyquaternium-52 | SOFCARE KG-101W-E (charge density 0.83 mmol/g) *1 (1% viscosity 2560 mPa s) active 2.4% | | | 0.320 | 0.120 | |
| | Polyquaternium-52 | SOFCARE KG-301W (charge density 1.84 mmol/g) *2 (1% viscosity 2070 mPa s) active 4.02% | | | | | |
| | Polyquaternium-37 | Ultragel 300 (charge density 4.81 mmol/g) *3 (1% viscosity 17650 mPa·s) | | | | | |
| (A') | Polyquaternium-22 | Merquat 295 (charge density 6.04 mmol/g) *4 (1% viscosity 5.6 mPa s) | 0.353 | 0.353 | | | |
| | Polyquaternium-55 | STYLEZE W17 (charge density unknown) *5 (1% viscosity 6.0 mPa·s) | | | | | 0.353 |
| (B1) | Sodium alginate | KIMICA ALGIN I-3-150 *6 (charge density 4.65 mmol/g, 1% viscosity 300 to 400 mPa·s) | | | 0.075 | 0.278 | 0.041 |
| | Sodium alginate | KIMICA ALGIN IL-2 *7 (charge density 4.65 mmol/g, 1% viscosity 20 to 50 mPa s) | | | | | |
| (B2) | (Acrylic acid/stearyl acrylate) copolymer | SOFCARE SA-37W *8 (charge density 8.88 mmol/g. Mw 20000) | | | | | |
| | Polyacrylic acid | Jurymer AC-10L *9 (charge density 13.88 mmol/g, Mw 20000 to 30000) | | | | | |
| | Polyacrylic acid | Polyacrylic acid (Mw 25000) *10 | 0.041 | | | | |
| | Polyacrylic acid | Polyacrylic acid (Mw 5000) *11 | | 0.041 | | | |
| (C) | Amino-modified silicone having a viscosity of 100 mm2/s or more and 5000 mm2/s or less | SF8457C *12 | | | 0.176 | 0.176 | 0.176 |
| | Polyglycerol-modified silicone having a viscosity of 100 mm2/s or more and 5000 mm2/s or less | SOFCARE GS-G *13 | | | | | |
| (C') | Aminopolyether-modified silicone having a viscosity of 100 mm2/s or more and 5000 mm2/s or less | Silicone SILSTYLE104 *14 | 0.12 | 0.12 | | | |
| (D) | (D1) Dimethylpolysiloxane having a viscosity of 1000 mm2/s or more and 10000 mm2/s or less | 5000cs silicone | | | 0.602 | 0.602 | 0.602 |
| | (D2) Dimethylpolysiloxane having a viscosity of 300000 mm2/s or more and 5000000 mm2/s or less | Highly polymerized silicone | | | 0.222 | 0.222 | 0.222 |
| | Dimethylpolysiloxane | KHS-3 *15 | 0.88 | 0.88 | | | |
| (G) | Polyethylene glycol | BLAUNON PEG-20000S *16 | | | 0.4 | 0.4 | 0.4 |
| | Polyvinylpyrrolidone | Luviskol K90 *17 | | | | | |
| (E) | Stearoxypropyldimethylamine | FARMIN DM-E80 *18 | | | | | |
| | Behenyltrimethylammonium chloride | QUARTAMIN 2285-E *19 | 1.2 | 1.2 | 1.2 | 1.2 | 1.2 |
| (F) | Cetanol | KALCOL 6870 *20 | 2.4 | 2.4 | 2.4 | 2.4 | 2.4 |
| (H) | Lactic acid | 90% Lactic acid *21 | Appropriate amount | Appropriate amount | Appropriate amount | Appropriate amount | Appropriate amount |
| Other Components | Dipropylene glycol | DPG-RF *22 | 4 | 4 | 4 | 4 | 4 |
| | Benzyl alcohol | | 0.3 | 0.3 | 0.3 | 0.3 | 0.3 |
| | Water | Ion exchange water | Balance | Balance | Balance | Balance | Balance |
| Total | | | 100 | 100 | 100 | 100 | 100 |

**Table 4 (continued)**

| (mass%) | | | Comparative Examples | | | | |
|---|---|---|---|---|---|---|---|
| | | | 7 | 8 | 9 | 10 | 11 |
| pH (25°C, at 20-fold dilution) | | | 3.4 | 3.4 | 3.4 | 3.4 | 3.4 |
| Content of cationic polymer (component (A) or (A')) in composition (mass%) | | | 0.353 | 0.353 | 0.320 | 0.120 | 0.353 |
| Content of anionic polymer (component (B)) in composition (mass%) | | | 0.041 | 0.041 | 0.075 | 0.278 | 0.041 |
| Total content of cationic polymer and anionic polymer in composition (mass%) | | | 0.394 | 0.394 | 0.395 | 0.398 | 0.394 |
| Cation (A) or (A')/anion (B1)/anion (B2) charge ratio (molar ratio) | | | 79/0/21 | 79/0/21 | 43/57/0 | 7/93/0 | Unknown |
| Mass ratio (A)/(B) (or (A')/(B)) | | | - | - | 4.28 | 0.43 | 8.61 |
| Content of component (B1) in component (B) (mass%) | | | 0.00 | 0.00 | 100.00 | 100.00 | 100.00 |
| Content of component (B2) in component (B) (mass%) | | | 100.00 | 100.00 | 000 | 000 | 000 |
| Mass ratio (B2)/{(B1) + (B2)} | | | 1.00 | 1.00 | 0.00 | 0.00 | 000 |
| Content of component (C) in composition (mass%) | | | 0.120 | 0.120 | 0.176 | 0.176 | 0.176 |
| Content of component (D) in composition (mass%) | | | 0.880 | 0.880 | 0.824 | 0.824 | 0.824 |
| Total content of component (C) and component (D) in composition (mass%) | | | 1.000 | 1.000 | 1.000 | 1.000 | 1.000 |
| Mass ratio (C)/(D) | | | 0.14 | 0.14 | 0.21 | 0.21 | 0.21 |
| Total content of components (A) to (F) in composition (mass%) | | | 4.64 | 4.64 | 4.99 | 5.00 | 4.64 |
| Content of component (G) in composition (mass%) | | | 0.00 | 0.00 | 0.40 | 0.40 | 0.40 |
| Mass ratio (G)/{(A) + (B)} (or (G)/{(A') + (B)}) | | | 0.00 | 000 | 1.01 | 1.01 | 1.02 |
| Effect | Combing force after hair conditioner treatment + two times of hair washing | Standard: 1900 g or less *blank = 2055 g | 1418 | 1291 | 1926 | 1961 | 1978 |
| | Combing force after hair conditioner treatment + four times of hair washing | | | | | | |
| | Emulsified state of hair conditioner after 2 days from blending | A: Aggregated particles cannot be visually recognized, and slippery when crushed | D | D | A | C | A |
| | | B: Aggregated particles cannot be visually recognized, but rough when crushed. | | | | | |
| | | C: Aggregated particles can be visually recognized, and are rough when crushed. | | | | | |
| | | D: Cd is separated | | | | | |
| | Moisture resistance of hair after treatment | Standard: less than 1.3 | 1.32 | 1.38 | 1.00 | 1.21 | 1.61 |

The components listed in the table are as follows. The blending amount (mass%) described in the table is an effective amount of each component.
<Component (A): Cationic Polymer>
   * Polyquaternium-52 (N,N-dimethylaminoethyl methacrylate diethyl sulfate-N, N-dimethylacrylamide-polyethylene glycol dimethacrylate copolymer), "SOFCARE KG-101W-E" manufactured by Kao Corporation
   *2 Polyquaternium-52 (N,N-dimethylaminoethyl methacrylate diethyl sulfate-N,N-dimethylacrylamide-polyethylene glycol dimethacrylate copolymer), "SOFCARE KG-301W" manufactured by Kao Corporation
   *3 Polyquaternium-37 (methacryloylethyltrimethylammonium chloride polymer), "Cosmedia Ultragel 300" manufactured by BASF Japan Ltd.
<Component (A'): Cationic Polymer Other Than Component (A)>
   *4 Polyquaternium-22 (dimethyldiallylammonium chloride-acrylic acid copolymer liquid), "Merquat 295" manufactured by Lubrizol Advanced Materials, Inc.
   *5 Polyquaternium-55 (polymer of quaternary ammonium salts obtained by the reaction of vinylpyrrolidone, dimethylaminopropylmethacrylamide and methacrylamidopropyllauryldimonium chloride), "STYLEZE W17" manufactured by Ashland Japan Ltd.
<Component (B1): Anionic Polymer>
   *6 Sodium alginate, "KIMICA ALGIN I-3-150" manufactured by Kimica Corporation
   *7 Sodium alginate, "KIMICAALGIN IL-2" manufactured by Kimica Corporation
<Component (B2): Anionic Polymer>
   *8 (Acrylate/stearyl acrylate) copolymer, "SOFCARE SA-37W " manufactured by Kao Corporation
   *9 Polyacrylic acid "Jurymer AC-10L" manufactured by TOAGOSEI CO., LTD.
   *10 Polyacrylic acid (Mw 25000), manufactured by FUJIFILM Wako Pure Chemical Industries, Ltd.
   *11 Polyacrylic acid (Mw 5000), manufactured by FUJIFILM Wako Pure Chemical Corporation
<Component (C): Modified Silicone>
   *12 Amino-modified silicone, "Silicone SF 8457C" manufactured by Dow Toray Co., Ltd.
   *13 Polyglycerol-modified silicone, "SOFCARE GS-G" manufactured by Kao Corporation
<Component (C'): Modified Silicone Other than Component (C)>
   * 14 Aminopolyether-modified silicone, "DOWSIL SILSTYLE104" manufactured by Dow Toray Co., Ltd.
<Component (D): Dimethylpolysiloxane>
   *15 Dimethylpolysiloxane "Silicone KHS-3" manufactured by Shin-Etsu Chemical Co., Ltd.
<Component (G): Nonionic Polymer>
   * 16: Polyethylene glycol, "BLAUNON PEG-20000S" manufactured by AOKI OIL INDUSTRIAL Co., Ltd., Mw: 20000
   *17 Polyvinylpyrrolidone, "Luviskol K90" manufactured by BASF Japan Ltd., Mw: 1200000
<Component (E): Cationic Surfactant>
   *18 Stearoxypropyldimethylamine, "FARMIN DM-E80" manufactured by Kao Corporation
   *19 Behenyltrimethylammonium chloride, "QUARTAMIN 2285-E" manufactured by Kao Corporation
<Component (F): Higher Alcohol>
   *20 Cetanol, "Kalcol 6870" manufactured by Kao Corporation
<Component (H): Acid>
   *21 90% Lactic acid, "PURAC ULTRAPURE90" manufactured from PURAC Thailand Ltd.
<Other>
   *22 Dipropylene glycol, "DPG-RF" manufactured by ADEKA Corporation

As shown in Tables 1 to 4, it can be seen that the compositions of the present examples can improve the effect of suppressing entanglement of hair and the durability thereof when used for hair treatment, can suppress spreading of hair under high humidity conditions, and have high stability. On the other hand, the cosmetic compositions of Comparative Examples were inferior in any one of the effect of suppressing entanglement of hair, the effect of improving the durability thereof, the effect of suppressing spreading of hair, and the stability.

### Industrial Applicability

According to the present invention, it is possible to provide a cosmetic composition or a fiber treatment composition for head decoration products that can improve the effect of improving the texture of an object to be treated, the durability of the effect, and the moisture resistance, can improve the effect of suppressing entanglement of hair or fibers for head decoration products and the durability thereof, can suppress the spread of hair under high humidity conditions, and has high stability.

## Claims

1. A cosmetic composition or a fiber treatment composition for head decoration products, the composition comprising:
(A) a cationic polymer having a viscosity when in a 1 mass% aqueous solution form at 30°C of 1000 mPa·s or more;
(B) an anionic polymer;
(C) one or more modified silicones selected from the group consisting of a polyglycerol-modified silicone and an amino-modified silicone;
(D) a dimethylpolysiloxane;
(E) a cationic surfactant; and
(F) a higher alcohol, wherein
a mass ratio [(A)/(B)] of the component (A) to the component (B) is 4.5 or more.

2. The composition according to claim 1, wherein the component (A) is a polymer containing a constituent unit represented by General Formula (1) described below: 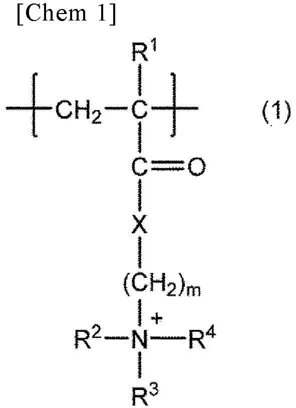 wherein R¹ is a hydrogen atom or a methyl group, and R² to R⁴ are each independently an alkyl group having 1 or more and 4 or less carbons, X is -O- or -NH-, and m is the number of 1 or more and 4 or less.

3. The composition according to claim 1 or 2, wherein the component (B) comprisies (B1) one or more selected from the group consisting of a crosslinked polymer containing a constituent unit derived from (meth)acrylic acid and an anionic polysaccharide.

4. The composition according to claim 3, wherein the component (B1) is alginic acid or a salt thereof.

5. The composition according to any one of claims 1 to 4, wherein the component (D) comprises:
(D1) a dimethylpolysiloxane having a viscosity at 25°C of 1000 mm²/s or more and 10000 mm²/s or less; and
(D2) a dimethylpolysiloxane having a viscosity at 25°C of 300000 mm²/s or more and 5000000 mm²/s or less.

6. The composition according to any one of claims 1 to 5, wherein the component (A) is one or more selected from the group consisting of a methacryloylethyltrimethylammonium chloride polymer and an N,N-dimethylaminoethylmethacrylic acid diethyl sulfate-N,N-dimethylacrylamide-polyethylene glycol dimethacrylate copolymer.

7. The composition according to any one of claims 1 to 6, further comprising (G) a nonionic polymer.

8. The composition according to any one of claims 3 to 7, wherein the component (B) further contains (B2) an anionic polymer other than the component (B1).

9. The composition according to claim 8, wherein the component (B2) is a non-crosslinked polymer having a molecular weight of 1000 or more and 30000 or less and containing a constituent unit derived from (meth)acrylic acid.

10. The composition according to any one of claims 1 to 9, wherein the mass ratio [(A)/(B)] of the component (A) to the component (B) contained in the composition is 20 or less.
